(19) Europäisches Patentamt
European Patent Office
Office européen des brevets

(11) EP 0 610 764 B1

(12) **EUROPÄISCHE PATENTSCHRIFT**

(45) Veröffentlichungstag und Bekanntmachung des Hinweises auf die Patenterteilung:
**12.05.1999 Patentblatt 1999/19**

(21) Anmeldenummer: **94101357.5**

(22) Anmeldetag: **31.01.1994**

(51) Int. Cl.$^6$: **A01N 47/12**
// (A01N47/12, 47:04, 37:34, 47:14, 47:26, 43:66, 59:20, 37:38, 43:32, 47:34, 47:12, 57:12, 37:46, 43:76, 43:40, 43:54, 37:50, 43:653, 43:82, 47:32)

(54) **Fungizide Wirkstoffkombinationen**
Fungicidal mixture
Combinaison d'agents fongicides

(84) Benannte Vertragsstaaten:
**BE CH DE ES FR GB GR IT LI NL PT**

(30) Priorität: **12.02.1993 DE 4304172**

(43) Veröffentlichungstag der Anmeldung:
**17.08.1994 Patentblatt 1994/33**

(73) Patentinhaber: **BAYER AG**
**51368 Leverkusen (DE)**

(72) Erfinder:
- **Dehne, Heinz-Wilhelm, Dr. habil.**
**D-40789 Monheim (DE)**
- **Brandes, Wilhelm, Dr.**
**D-42799 Leichlingen (DE)**
- **Kuck, Karl-Heinz, Dr.**
**D-40764 Langenfeld (DE)**
- **Seitz, Thomas, Dr.**
**D-40764 Langenfeld (DE)**

(56) Entgegenhaltungen:
**EP-A- 0 472 996**
**EP-A- 0 493 683**
**EP-A- 0 550 788**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 610 764 B1

Printed by Xerox (UK) Business Services
2.16.7/3.6

## Beschreibung

Die vorliegende Anmeldung betrifft neue Wirkstoffkombinationen, die aus Valinamid-Derivaten einerseits und weiteren bekannten fungiziden Wirkstoffen andererseits bestehen und sehr gut zur Bekämpfung von phytopathogenen Pilzen geeignet sind.

Es ist bereits bekannt, daß Valinamid-Derivate fungizide Eigenschaften besitzt (vgl. EP-A 472 996). Die Wirksamkeit dieses Stoffes ist gut; sie läßt jedoch bei niedrigen Aufwandmengen in manchen Fällen zu wünschen übrig.

Ferner ist schon bekannt, daß zahlreiche Azol-Derivate, aromatische Carbonsäure-Derivate, Morpholin-Verbindungen und andere Heterocyclen zur Bekämpfung von Pilzen eingesetzt werden können (vgl. K.H. Büchel "Pflanzenschutz und Schädlingsbekämpfung" Seiten 87, 136, 140, 141 und 146 bis 153, Georg Thieme Verlag, Stuttgart 1977). Die Wirkung der betreffenden Stoffe ist aber bei niedrigen Aufwandmengen nicht immer befriedigend.

Es wurde nun gefunden, daß die neuen Wirkstoffkombinationen aus Valinamid-Derivaten der allgemeinen Formel (I)

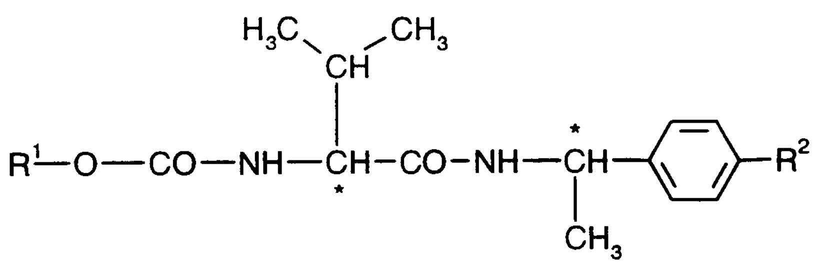

(I)

in welcher

$R^1$ für i-Propyl oder s-Butyl und

$R^2$ für Chlor, Methyl, Ethyl oder Methoxy steht, und

(A) Dichlofluanid der Formel

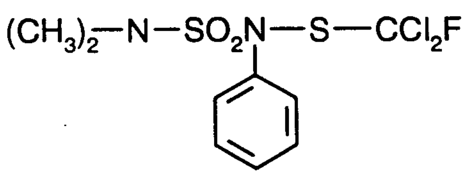

(II)

und/oder
(B) Tolylfluanid der Formel

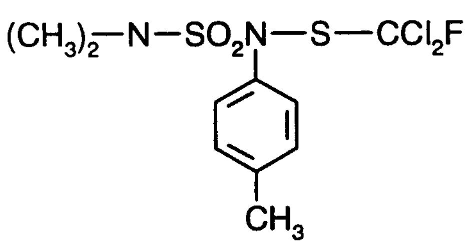

(III)

und/oder
(C) Tetrachlor-isophthalo-dinitril der Formel

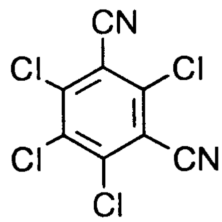

(IV)

(CHLOROTHALONIL)

und/oder

(D) Propineb der Formel

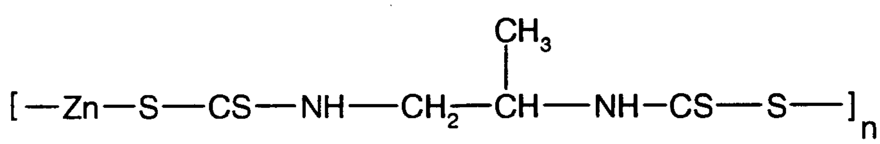

(V)

und/oder

(E) Tetramethyl-thiuram-disulfid der Formel

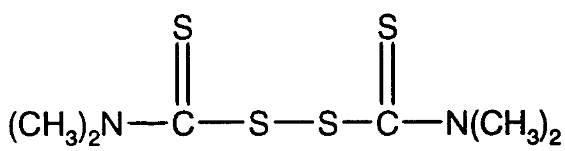

(VI)

(THIRAM)

und/oder

(F) Mancozeb der Formel

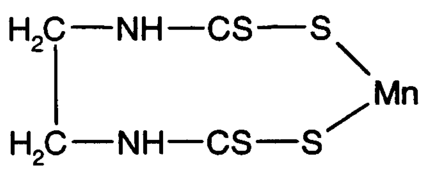

(VI)

und/oder

(G) Dyrene der Formel

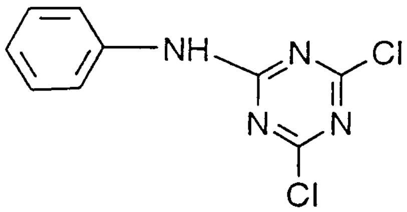

(VII)

und/oder

(H) Kupfer-Oxychloride

und/oder

(I) Captan der Formel

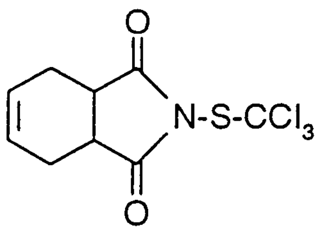

(VIII)

und/oder

(K) einem Morpholin-Derivat der Formel

(Dimetomorph)

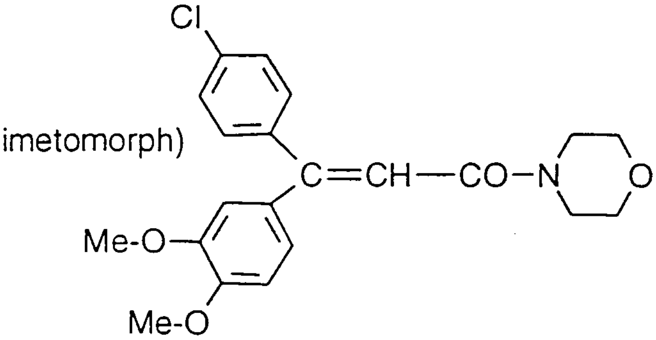

(IX)

und/oder

(L) Dithianon der Formel

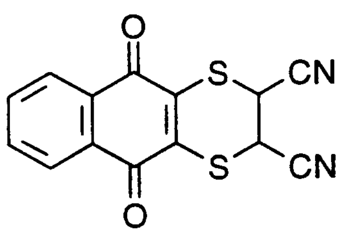

(X)

und/oder

(M) Phaltan der Formel

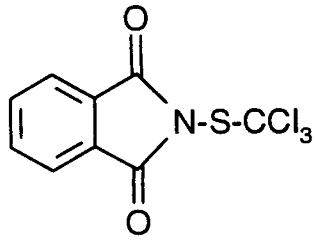

(XI)

und/oder

(N) Cymoxanil der Formel

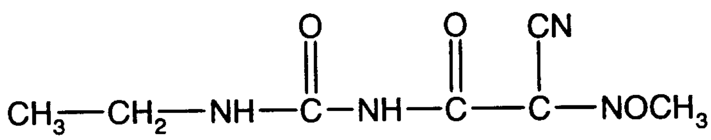

(XII)

und/oder

(O) Propamocarb der Formel

$$(CH_3)_2NCH_2\text{-}CH_2\text{-}CH_2\text{-}NHCO\text{-}OCH_2CH_3 \quad (XIII)$$

bzw. dessen Hydrochlorid

und/oder

(P)Fosetyl der Formel

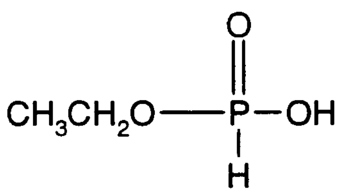

(XIV)

bzw. dessen Aluminium-Addukt

und/oder

(Q) Metalaxyl der Formel

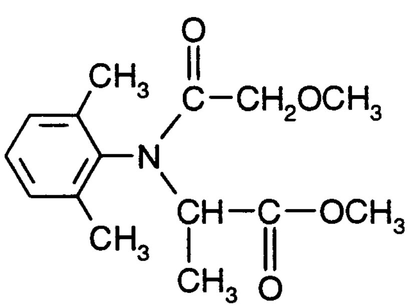

(XV)

und/oder

(R) Oxadixyl der Formel

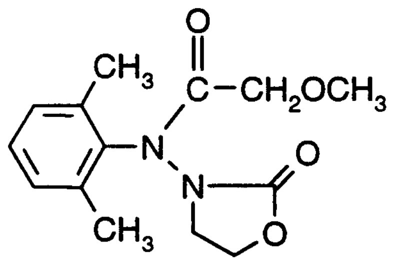

(XVI)

und/oder

(S) Fluazinam der Formel

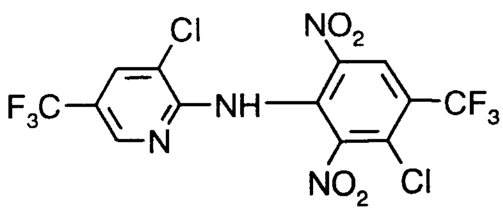

(XVII)

und/oder

(T) Methoxyacrylate wie

Methyl-(E)-2-{2-[6-(2-cyanophenoxy)pyrimidin-4-yloxy]phenyl )-3-methoxyacrylat der Formel

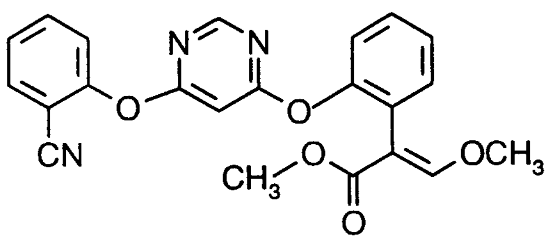

(XVIII)

und/oder

(U) Methoximinoacetate wie

Methyl-(E)-methoximino[α-(o-tolyloxy)-o-tolyl]acetat der Formel

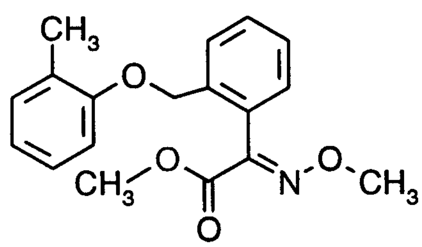

(XIX)

und/oder

(V) Furalaxyl ≙ Methyl-N-(2-furoyl)-N-(2,6-xylyl)-alanit

und/oder

(W) Azole der Formel

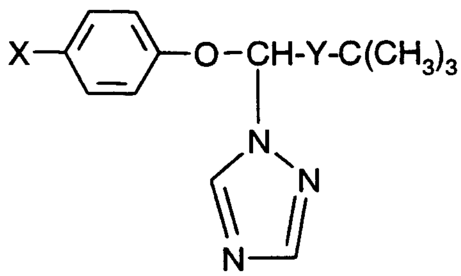

(XX)

I X = Cl, Y = -CH(OH)- (Triadimenol)

II X = 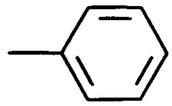 , Y = -CH(OH)- (Bitertanol)

III X = Cl, Y = -CO- (Triadimefon)

IV Tebuconazole der Formel

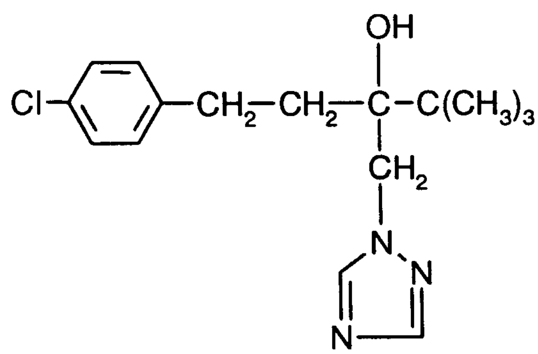

und/oder

(X) Etridiazole ≙ 3-Trichlormethyl-5-ethoxy-1,2,4-thiadiazol

und/oder

(Y) Pencycuron ≙ 1-(4-Chlorbenzyl)-1-cyclopentyl-3-phenylharnstoff sehr gute fungizide Eigenschaften besitzen.

Überraschenderweise ist die fungizide Wirkung der erfindungsgemäßen Wirkstoffkombination wesentlich höher als die Summe der Wirkungen der einzelnen Wirkstoffe. Es liegt ein nicht vorhersehbarer echter synergistischer Effekt vor und nicht nur eine Wirkungsergänzung.

Aus der Strukturformel für die Wirkstoffe der Formel (I) ist ersichtlich, daß die Verbindungen zwei asymmetrisch substituierte Kohlenstoffatome aufweisen. Das Produkt kann daher als Gemisch von verschiedenen Isomeren oder auch in Form eines einzigen Isomeren vorliegen.

Bevorzugte Verbindungen der Formel (I) sind Verbindungen in denen der Aminosäureteil aus i-Propyloxycarbonyl-L-valin oder sec-Butoxycarbonyl-L-valin gebildet wird und der Phenethylaminteil entweder racemisch ist oder die S(-)Konfiguration, insbesondere aber die R(+)-Konfiguration aufweist.

Besonders bevorzugte Verbindungen der Formel (I) sind die Verbindungen in denen $R^1$ für i-Propyl steht.

Insbesondere seien die Verbindungen [2-Methyl-1-[[[-1-(4-chlorphenyl)ethyl]-amino]carbonyl]-propyl]-carbaminsäure-1-methylethylester der Formel (Ia)

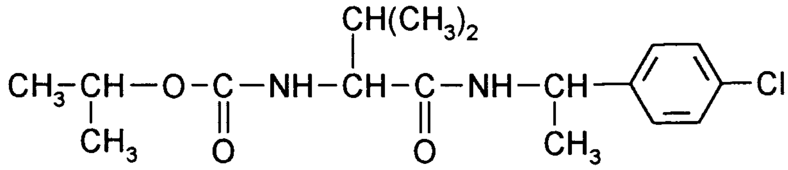

(Ia)

[2-Methyl-1-[[[-1-(4-methylphenyl)ethyl]amino)carbonyl]-propyl]-carbaminsäure-1-methylethylester der Formel (Ib)

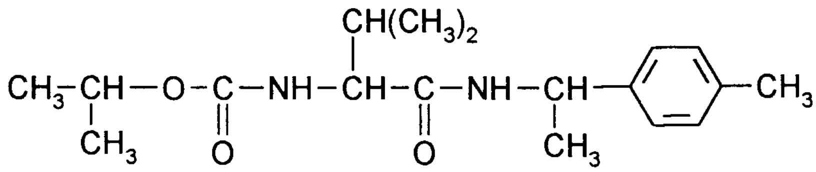

(Ib)

[2-Methyl-1-[[[-1-(4-ethylphenyl)ethyl]amino]carbonyl]-propyl]-carbaminsäure-1-methylethylester der Formel (Ic)

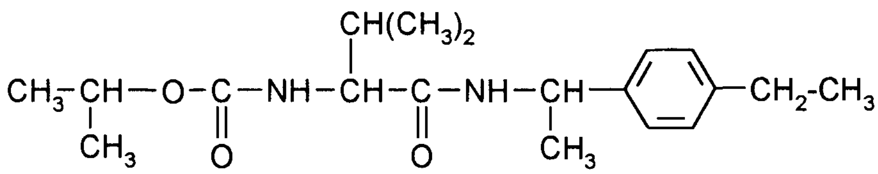

(Ic)

und [2-Methyl-1-[[[-1-(4-methoxyphenyl)ethyl]amino)carbonyl]-propyl]-carbaminsäure-1-methylethylester der Formel (Id)

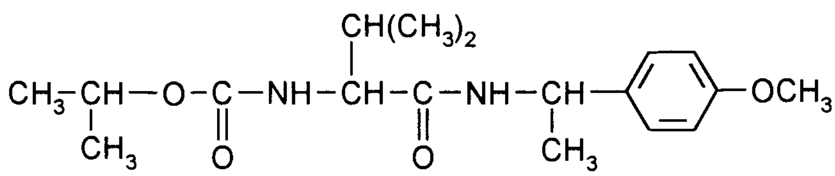

(Id)

und deren Isomere, wie oben erwähnt, genannt.

Die Wirkstoffe der Formel (I) sind bekannt (vgl. EP-A-472 996).

Die in den erfindungsgemäßen Kombinationen außerdem vorhandenen fungiziden Wirkstoffe sind ebenfalls bekannt. Im einzelnen werden die Wirkstoffe in folgenden Publikationen beschrieben:

(A): K.H. Büchel "Pflanzenschutz und Schädlingsbekämpfung", Seite 141, Georg Thieme Verlag, Stuttgart 1977;
(B): K.H. Büchel, loc. cit., Seite 141(G) S. 153
(C): K.H. Büchel, loc. cit., Seite 146 (H) S. 122
(D): K.H. Büchel, loc. cit., Seite 138 (I) S. 132, 140
(E): K.H. Büchel, loc. cit., Seite 136 (K) EP-A-219 756
(F): K.H. Büchel, loc. cit., Seite 137 (L) S. 145
(G): K.H. Büchel, loc. cit., Seite 153
(H): K.H. Büchel, loc. cit., Seite 122
(I): K.H. Büchel, loc. cit., Seite 132
(K): EP-A-219 756
(L): K.H. Büchel, loc. cit., Seite 145
(M): K.H. Büchel, loc. cit., Seite 140
(O): DE 1 567 169

(P): FR 2 254 276
(Q): GB-1 500 581
(R): GB-2 058 059
(S): EP-031 257
(T): Brighton Crop Protection Conference (1992) 5-6, 435-37
(U): Brighton Crop Protection Conference (1992) 5-2, 403-05
(V): GB-1 448 810
(W): EP-0 040 345, DE 2 324 010, DE 2 201 063
(X): US 3 260 588
(Y): DE 2 732 257

Die erfindungsgemäßen Wirkstoffkombinationen enthalten neben mindestens einem Wirkstoff der Formel (I) mindestens einen Wirkstoff von den Verbindungen der Gruppen (A) bis (Y). Sie können darüber hinaus auch weitere fungizid wirksame Zumischkomponenten enthalten.

Wenn die Wirkstoffe in den erfindungsgemäßen Wirkstoffkombinationen in bestimmten Gewichtsverhältnissen vorhanden sind, zeigt sich der synergistische Effekt besonders deutlich. Jedoch können die Gewichtsverhältnisse der Wirkstoffe in den Wirkstoffkombinationen in einem relativ großen Bereich variiert werden. Im allgemeinen entfallen auf 1 Gewichtsteil an Wirkstoff der Formel (I).

| | |
|---|---|
| 1 bis | 50 Gewichtsteile, vorzugsweise |
| 2,5 bis | 10 Gewichtsteile an Wirkstoff aus der Gruppe (A) |
| | |
| 1 bis | 50 Gewichtsteile, vorzugsweise |
| 2,5 bis | 10 Gewichtsteile an Wirkstoff aus der Gruppe (B) |
| | |
| 1 bis | 50 Gewichtsteile, vorzugsweise |
| 1 bis | 10 Gewichtsteile an Wirkstoff aus der Gruppe (C) |
| | |
| 1 bis | 50 Gewichtsteile, vorzugsweise |
| 5 bis | 20 Gewichtsteile an Wirkstoff aus der Gruppe (D) |
| | |
| 1 bis | 200 Gewichtsteile, vorzugsweise |
| 1 bis | 100 Gewichtsteile an Wirkstoff aus der Gruppe (E) |

| | |
|---|---|
| 1 bis | 50 Gewichtsteile, vorzugsweise |
| 5 bis | 20 Gewichtsteile an Wirkstoff aus der Gruppe (F) |
| 1 bis | 200 Gewichtsteile, vorzugsweise |
| 1 bis | 100 Gewichtsteile an Wirkstoff aus der Gruppe (G) |
| 1 bis | 200 Gewichtsteile, vorzugsweise |
| 1 bis | 50 Gewichtsteile an Wirkstoff aus der Gruppe (H) |
| 1 bis | 100 Gewichtsteile, vorzugsweise |
| 1 bis | 50 Gewichtsteile an Wirkstoff aus der Gruppe (I) |
| 0,5 bis | 10 Gewichtsteile, vorzugsweise |
| 1 bis | 5 Gewichtsteile an Wirkstoff aus der Gruppe (K) |
| 1 bis | 50 Gewichtsteile, vorzugsweise |
| 1 bis | 20 Gewichtsteile an Wirkstoff aus der Gruppe (L) |
| 1 bis | 50 Gewichtsteile, vorzugsweise |
| 1 bis | 20 Gewichtsteile an Wirkstoff aus der Gruppe (M) |
| 0,5 bis | 20 Gewichtsteile, vorzugsweise |
| 1 bis | 10 Gewichtsteile an Wirkstoff aus der Gruppe (N) |
| 1 bis | 100 Gewichtsteile, vorzugsweise |
| 1 bis | 50 Gewichtsteile an Wirkstoff aus der Gruppe (O) |
| 1 bis | 50 Gewichtsteile, vorzugsweise |
| 1 bis | 20 Gewichtsteile an Wirkstoff aus der Gruppe (P) |
| 0,5 bis | 20 Gewichtsteile, vorzugsweise |
| 1 bis | 10 Gewichtsteile an Wirkstoff aus der Gruppe (Q) |
| 0,5 bis | 20 Gewichtsteile, vorzugsweise |
| 1 bis | 10 Gewichtsteile an Wirkstoff aus der Gruppe (R) |

| | |
|---|---|
| 1 bis | 20 Gewichtsteile, vorzugsweise |
| 1 bis | 10 Gewichtsteile an Wirkstoff aus der Gruppe (S) |

| | |
|---|---|
| 0,5 bis | 20 Gewichtsteile, vorzugsweise |
| 1 bis | 10 Gewichtsteile an Wirkstoff aus der Gruppe (T) |

| | |
|---|---|
| 0,5 bis | 20 Gewichtsteile, vorzugsweise |
| 1 bis | 10 Gewichtsteile an Wirkstoff aus der Gruppe (U) |

| | |
|---|---|
| 0,5 bis | 20 Gewichtsteile, vorzugsweise |
| 1 bis | 10 Gewichtsteile an Wirkstoff aus der Gruppe (V) |

| | |
|---|---|
| 0,5 bis | 20 Gewichtsteile, vorzugsweise |
| 1 bis | 10 Gewichtsteile an Wirkstoff aus der Gruppe (W) |

| | |
|---|---|
| 0,5 bis | 20 Gewichtsteile, vorzugsweise |
| 1 bis | 10 Gewichtsteile an Wirkstoff aus der Gruppe (X) |

| | |
|---|---|
| 0,5 bis | 20 Gewichtsteile, vorzugsweise |
| 1 bis | 10 Gewichtsteile an Wirkstoff aus der Gruppe (Y). |

Die erfindungsgemäßen Wirkstoffkombinationen besitzen sehr gute fungizide Eigenschaften und lassen sich vor allem zur Bekämpfung von phytopathogenen Pilzen, wie Plasmodiophoromycetes, Oomycetes, Chytridiomycetes, Zygomycetes, Ascomycetes, Basidiomycetes, Deuteromycetes usw. einsetzen.

Die erfindungsgemäßen Wirkstoffkombinationen eignen sich besonders gut zur protektiven Bekämpfung von Phytophthora infestans und Alternaria spec. an Tomaten und Kartoffel, sowie Plasmopara viticola an Weinrebe.

Die gute Pflanzenverträglichkeit der Wirkstoffkombinationen in den zur Bekämpfung von Pflanzenkrankheiten notwendigen Konzentrationen erlaubt eine Behandlung von oberirdischen Pflanzenteilen, von Pflanz- und Saatgut, und des Bodens.

Die erfindungsgemäßen Wirkstoftkombinationen können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe bzw. der Wirkstoffkombinationen mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier- und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycol-ether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die Wirkstoffe der Formel (I) und die erfindungsgemäßen Wirkstoffkombinationen können in den Formulierungen in Mischung mit anderen Wirkstoffen vorliegen, wie Fungizide, Insektizide, Akarizide und Herbizide, sowie in Mischungen mit Düngemitteln bder Pflanzenwachstumsregulatoren.

Für solche Mischungen kommen beispielsweise infrage:

**Fungizide:**

2-Aminobutan; 2-Anilino-4-methyl-6-cyclopropyl-pyrimidin; 2',6'-Dibromo-2-methyl-4'-trifluoromethoxy-4'-trifluoromethyl-1,3-thizole-5-carboxanilid; 2,6-Dichloro-N-(4-trifluoromethylbenzyl)benzamid; (E)-2-Methoxyimino-N-methyl-2-(2-phenoxyphenyl) acetamid; 8-Hydroxyquinolinsulfat; Methyl-(E)-2-{2-[6-(2-cyanophenoxy)pyrimidin-4-yloxy]phenyl}-3-methoxyacrylat; Methyl-(E)-methoximino [alpha-(o-tolyloxy)-o-tolyl] acetat; 2-Phenylphenol (OPP), Aldimorph, Ampropylfos, Anilazin, Azaconazol,
Benalaxyl, Benbdanil, Benomyl, Binapacryl, Biphenyl, Bitertanol, Blasticidin-S, Bromuconazole, Bupirimate, Buthiobate,
Calciumpolysulfid, Captafol, Captan, Carbendazim, Carboxin, Chinomethionat (Quinomethionat), Chloroneb, Chloropicrin, Chlorothalonil, Chlozolinat, Cufraneb, Cymoxanil, Cyproconazole, Cyprofuram,
Dichlorophen, Diclobutrazol, Diclofluanid Diclomezin, Dicloran, Diethofencarb, Difenoconazol, Dimethirimol, Dimethomorph, Diniconazol, Dinocap, Diphenylamin, Dipyrithion, Ditalimfos, Dithianon, Dbdin, Drazoxolon,
Edifenphos, Epoxyconazole, Ethirimol, Etridiazol,
Fenarimol, Fenbuconazole, Fenfuram, Fenitropan, Fenpiclonil, Fenpropidin, Fenpropimorph, Fentinacetate, Fentinhydroxyd, Ferbam, Ferimzone, Fluazinam, Fludioxonil, Fluoromide, Fluquinconazole, Flusilazole, Flusulfamide, Flutolanil, Flutriafol, Folpet, Fosetyl-Aluminium, Fthalide, Fuberidazol, Furalaxyl, Furmecyclox,
Guazatine,
Hexachlorobenzol, Hexaconazol, Hymexazol,
Imazalil, Imibenconazol, Iminoctadin, Iprobenfos (IBP), Iprodion, Isoprothiolan,
Kasugamycin, Kupfer-Zubereitungen, wie: Kupferhydroxid, Kupfernaphthenat, Kupferoxychlorid, Kupfersulfat, Kupferoxid, Oxin-Kupfer and Bordeaux-Mischung,
Mancopper, Mancozeb, Maneb, Mepanipyrim, Mepronil, Metalaxyl, Metconazol, Methasulfocarb, Methfuroxam, Metiram, Metsulfovax, Myclobutanil,
Nickel dimethyldithiocarbamat, Nitrothal-isopropyl, Nuarimol,
Ofurace, Oxadixyl, Oxamocarb, Oxycarboxin,
Pefurazoat, Penconazol, Pencycuron, Phosdiphen, Pimaricin, Piperalin, Polyoxin, Probenazol, Prochloraz, Procymidon, Propamocarb, Propiconazole, Propineb, Pyrazophos, Pyrifenox, Pyrimethanil, Pyroquilon,
Quintozen (PCNB),
Schwefel und Schwefel-Zubereitungen,
Tebuconazol, Tecloftalam, Tecnazen, Tetraconazol, Thiabendazol, Thicyofen, Thiophanat-methyl, Thiram, Tolclophos-methyl, Tolylfluanid, Triadimefon, Triadimenol, Triazoxid, Trichlamid, Tricyclazol, Tridemorph, Triflumizol, Triforin, Triticonazol,
Validamycin A, Vinclozolin,

Zineb, Ziram

**Bakterizide:**

Bronopol, Dichlorophen, Nitrapyrin, Nickel Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

**Insektizide / Akarizide / Nematizide:**

Abamectin, Abamectin, AG 303 630, Acephat, Acrinathrin, Alanycarb, Aldicarb, Alphamethrin, Amitraz, Avermectin, AZ 60541, Azadirachtin, Azinphos A, Azinphos M, Azocyclotin,
Bacillus thuringiensis, Bendiocarb, Benfuracarb, Bensultap, Betacyluthrin, Bifenthrin, BPMC, Brofenprox, Bromophos A, Bufencarb, Buprofezin, Butocarboxin, Butylpyridaben,
Cadusafos, Carbaryl, Carbofuran, Carbophenothion, Carbosulfan, Cartap, CGA 157 419, CGA 184699, Chloethocarb, Chlorethoxyfos, Chloretoxyfos, Chlorfenvinphos, Chlorfluazuron, Chlormephos, Chlorpyrifos, Chlorpyrifos M, Cis-Resmethrin, Clocythrin, Clofentezin, Cyanophos, Cycloprothrin, Cyfluthrin, Cyhalothrin, Cyhexatin, Cypermethrin, Cyromazin,
Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat,
Dimethylvinphos, Dioxathion, Disulfoton,
Edifenphos, Emamectin, Esfenvalerat, Ethiofencarb, Ethion, Ethofenprox, Ethoprophos, Etofenprox, Etrimphos,
Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Flucycloxuron, Flucythrinat, Flufenoxuron, Flufenprox, Fluvalinate, Fonophos, Formothion, Fosthiazat, Fubfenprox, Furathiocarb,
HCH, Heptenophos, Hexaflumuron, Hexythiazox,
Imidacloprid, Iprobenfos, Isazophos, Isofenphos, Isoprocarb, Isoxathion, Ivemectin,
Lamda-cyhalothrin, Lufenuron,
Malathion, Mecarbam, Mervinphos, Mesulfenphos, Metaldehyd, Methacrifos, Methamidophos, Methidathion, Methiocarb, Methomyl, Metolcarb, Milbemectin, Monocrotophos, Moxidectin,
Naled, NC 184, NI 25, Nitenpyram
Omethoat, Oxamyl, Oxydemethon M, Oxydeprofos,
Parathion A, Parathion M, Permethrin, Phenthoat, Phorat, Phosalon, Phosmet, Phosphamdon, Phoxim, Pirimicarb, Pirimiphos M, ,Primiphos A, Profenofos, Profenophos, Promecarb, Propaphos, Propoxur, Prothiofos, Prothiophos, Prothoat, Pymetrozin, Pyrachlophos, Pyraclofos, Pyraclophos, Pyradaphenthion, Pyresmethrin, Pyrethrum, Pyridaben, Pyrimidifen, Pyriproxifen,
Quinalphos,
RH 5992,
Salithion, Sebufos, Silafluofen, Sulfotep, Sulprofos,
Tebufenozid, Tebufenpyrad, Tebupirimphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflumuron, Trimethacarb,
Vamidothion, XMC, Xylylcarb, YI 5301 / 5302, Zetamethrin.

Die Wirkstoffkombinationen können als solche, in Form ihrer Formulierungen oder den daraus bereiteten Anwendungsformen, wie gebrauchsfertige Lösungen, emulgierbare Konzentrate, Emulsionen, Suspensionen, Spritzpulver, lösliche Pulver und Granulate, angewendet werden. Die Anwendung geschieht in üblicher Weise, z.B. durch Gießen, Verspritzen, Versprühen, Verstreuen, Verstreichen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001Gew.-%, vorzugsweise zwischen 0,5 und 0,001%.

Bei der Saatgutbehandlung werden im allgemeinen Wirkstoffmengen von 0,001 bis 50g je Kilogramm Saatgut, vorzugsweise 0,01 bis 10g benötigt.

Bei Behandlung des Bodens sind Wirkstoffkonzentrationen von 0,00001 bis 0,1Gew.-%, vorzugsweise von

0,0001 bis 0,02Gew.-%, am Wirkungsort erforderlich.

Die gute fungizide Wirkung der erfindungsgemäßen Wirkstoffkombinationen geht aus den nachfolgenden Beispielen hervor. Während die einzelnen Wirkstoffe in der fungiziden Wirkung Schwächen aufweisen, zeigen die Kombinationen eine Wirkung, die über eine einfache Wirkungssummierung hinausgeht.

Ein synergistischer Effekt liegt bei Fungiziden immer dann vor, wenn die fungizide Wirkung der Wirkstoffkombinationen größer ist als die Summe der Wirkungen der einzeln applizierten Wirkstoffe.

Die zu erwartende Wirkung für eine gegebene Kombination zweier Wirkstoffe kann (vgl. Colby, S.R., "Calculating Synergistic and Antagonistic Responses of Herbicide Combinations", Weeds 15, Seiten 20-22, 1967) wie folgt berechnet werden:

Wenn

X den Wirkungsgrad, ausgedrückt in % der unbehandelten Kontrolle, beim Einsatz des Wirkstoffes A in einer Konzentration von m ppm,

Y den Wirkungsgrad, ausgedrückt in % der unbebandelten Kontrolle, beim. Einsatz des Wirkstoffes B in einer Konzentration von m ppm,

E den erwarteten Wirkungsgrad, ausgedrückt in % der unbebandelten Kontrolle, beim Einsatz des Wirkstoffes A und B in einer Konzentrationen von m und n ppm bedeutet,
dann ist

$$E = X + Y - \frac{X \cdot N}{100} .$$

Ist die tatsächliche fungizide Wirkung größer als berechnet, so ist die Kombination in ihrer Wirkung überadditiv, d.h. es liegt ein synergistischer Effekt vor. In diesem Fall muß der tatsächlich beobachtete Wirkungsgrad größer sein als der aus der oben angeführten Formel errechnete Wert für den erwarteten Wirkungsgrad (E):

## Patentansprüche

1. Synergistische wirkende Kombination von Verbindungen der Formel (I)

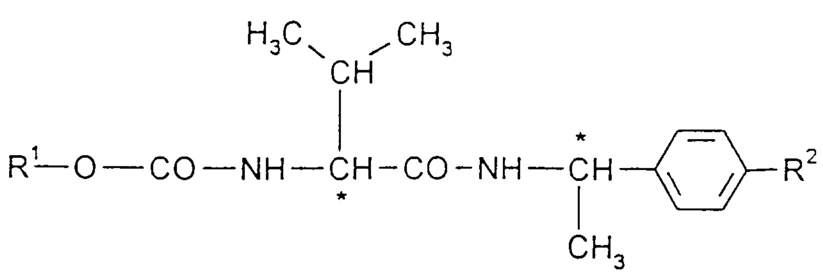

(I)

in welcher

$R^1$ für i-Propyl oder s-Butyl und

$R^2$ für Chlor, Methyl, Ethyl oder Methoxy steht,
und

(A) Dichlofluanid der Formel

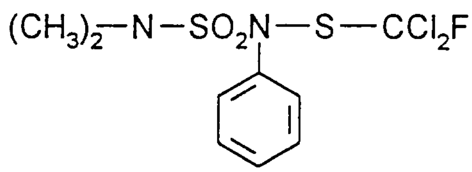

(II)

und/oder

(B) Tolylfluanid der Formel

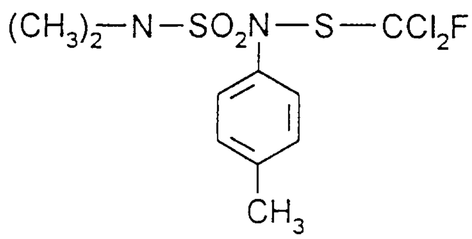

(III)

und/oder

(C) Tetrachlor-isophthalo-dinitril der Formel

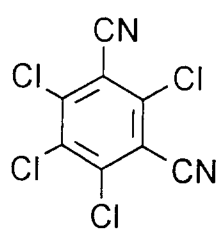

(IV)

(CHLOROTHALONIL)

und/oder

(D) Propineb der Formel

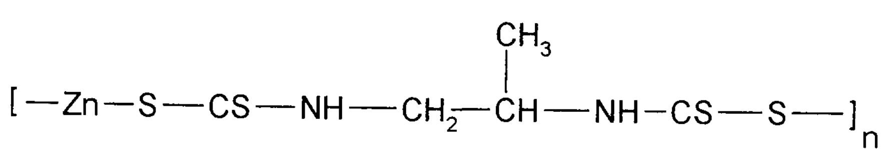

(V)

und/oder

(E) Tetramethyl-thiuram-disulfid der Formel

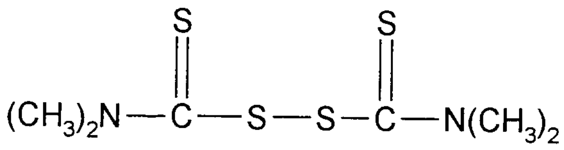

(VI)

(THIRAM)

und/oder

(F) Mancozeb der Formel

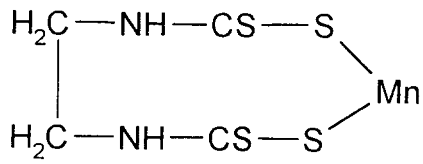

(VI)

und/oder

(G) Dyrene der Formel

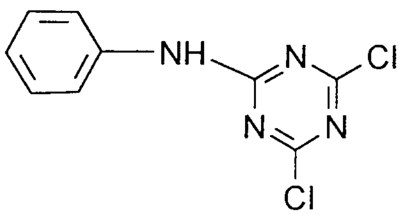

(VII)

und/oder

(H) Kupfer-Oxychloride
und/oder

(I) Captan der Formel

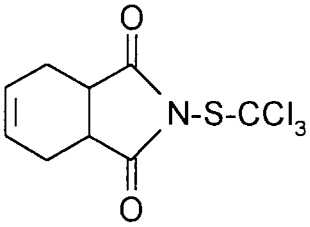

(VIII)

und/oder

(K) einem Morpholin-Derivat der Formel

(Dimetomorph)

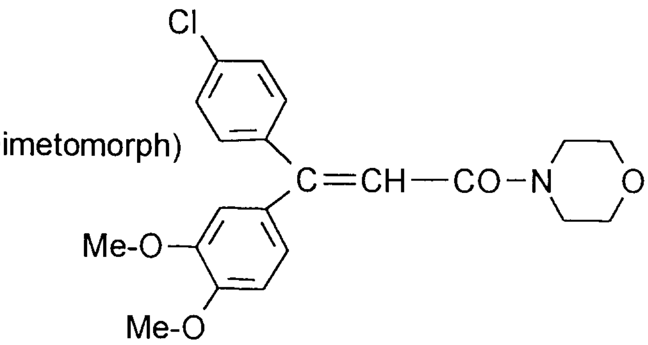

(IX)

und/oder

(L) Dithianon der Formel

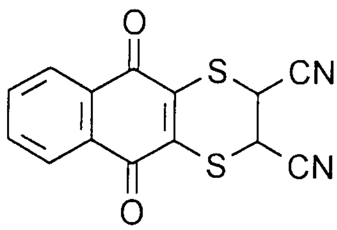

(X)

und/oder

(M) Phaltan der Formel

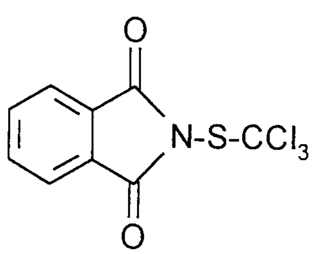

(XI)

und/oder

(N) Cymoxanil der Formel

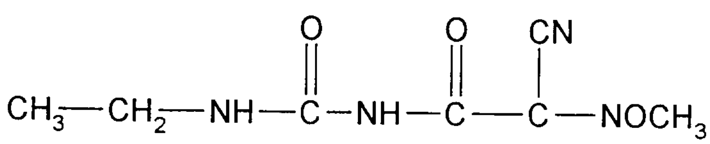

(XII)

und/oder

(O) Propamocarb der Formel

$$(CH_3)_2NCH_2\text{-}CH_2\text{-}CH_2\text{-}NHCO\text{-}OCH_2CH_3 \quad (XIII)$$

bzw. dessen Hydrochlorid
und/oder

(P) Fosetyl der Formel

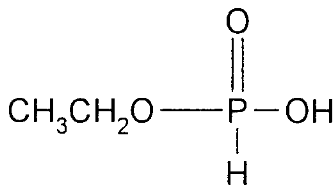

(XIV)

bzw. dessen Aluminium-Addukt und/oder

(Q) Metalaxyl der Formel

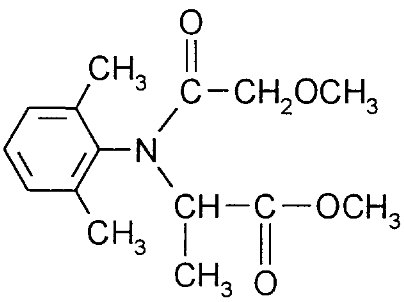

(XV)

und/oder

(R) Oxadixyl der Formel

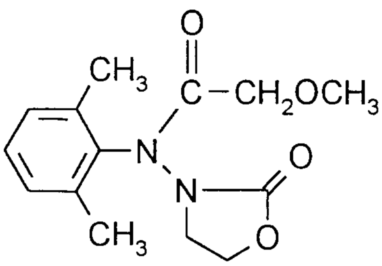

(XVI)

und/oder

(S) Fluazinam der Formel

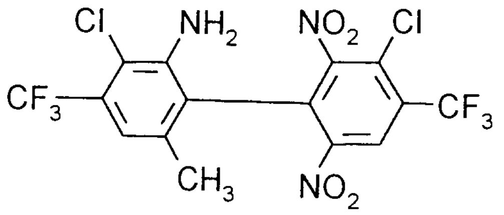

(XVII)

und/oder

(T) Methoxyacrylate wie
Methyl-(E)-2-{2-[6-(2-cyanophenoxy)pyrimidin-4-yloxy]phenyl}-3-methoxyacrylat der Formel

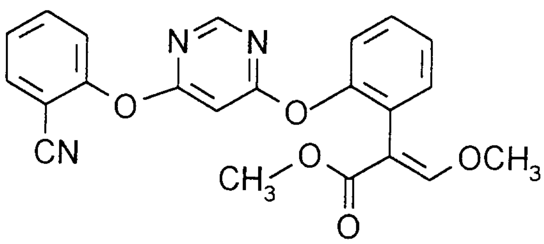

(XVIII)

und/oder

(U) Methoximinoacetate wie
Methyl-(E)-methoximino[$\alpha$-(o-tolyloxy)-o-tolyl]acetat der Formel

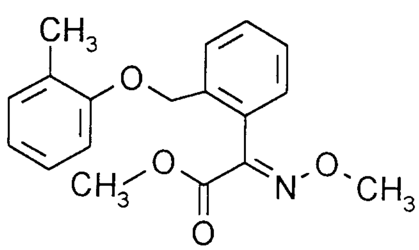

(XIX),

(V) Furalaxyl ≙ Methyl-N-(2-furoyl)-N-(2,6-xylyl)-alanit
und/oder

(W) Azole der Formel

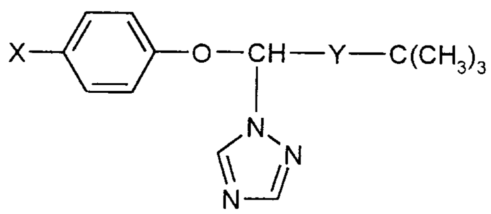

(XX)

I X = Cl, Y = -CH(OH)- (Triadimenol)

II X = 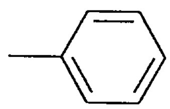 , Y = -CH(OH)- (Bitertanol)

III X = Cl, Y = -CO- (Triadimefon)

IV Tebuconazole der Formel

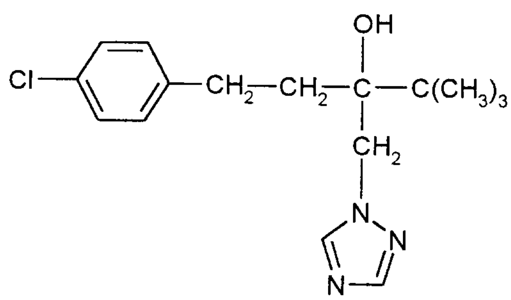

und/oder

(X) Etridiazole ≙ 3-Trichlormethyl-5-ethoxy-1,2,4-thiadiazol
und/oder

(Y) Penaycuron ≙ 1-(4-Chlorbenzyl)-1-cyclopentyl-3-phenylharnstoff.

**2.** Synergistisch wirkende Kombination von Verbindungen der Formel (Ib)

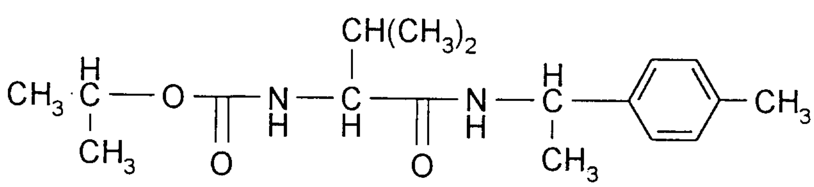

(Ib)

mit

(A) Dichlofluanid der Formel (II)

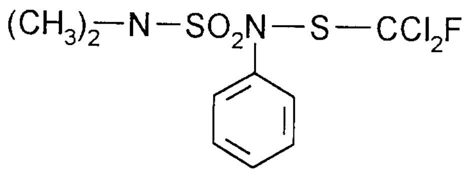

(II).

3. Synergistisch wirkende Kombination von Verbindungen der Formel (Ib)

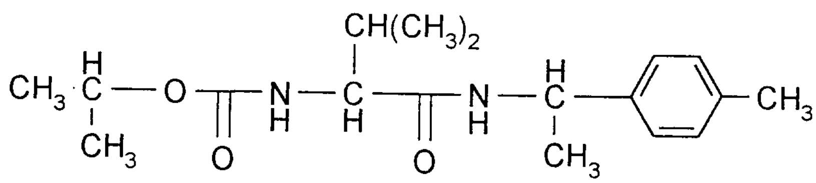

(Ib)

mit

(B) Tolylfluanid der Formel

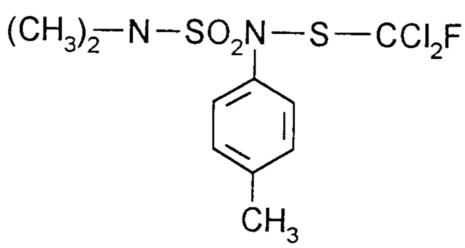

(III).

4. Synergistisch wirkende Kombination von Verbindungen der Formel (Ib)

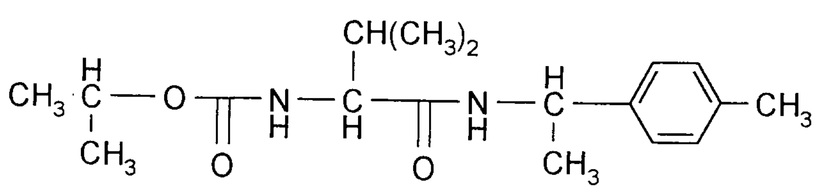

(Ib)

mit

(C) Tetrachlor-isophthalo-dinitril der Formel

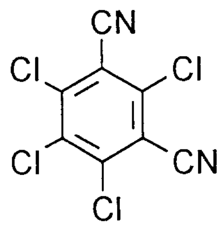

(IV)

(CHLOROTHALONIL)

**5.** Synergistisch wirkende Kombination von Verbindungen der Formel (Ib)

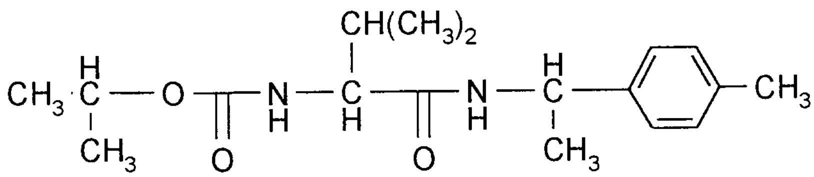

(Ib)

mit

(D) Propineb der Formel

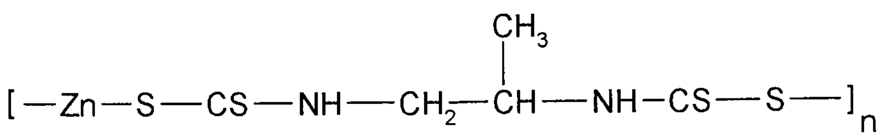

(V)

**6.** Synergistisch wirkende Kombination von Verbindungen der Formel (Ib)

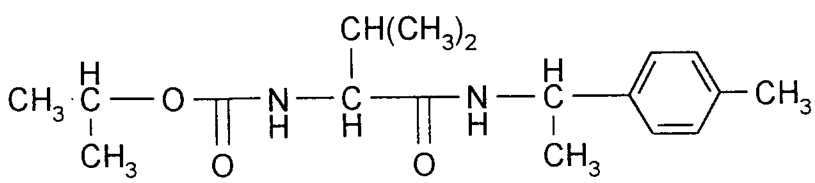

(Ib)

mit

(E) Tetramethyl-thiuram-disulfid der Formel

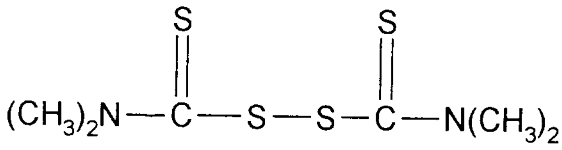 (VI)

(THIRAM)

**7.** Synergistisch wirkende Kombination von Verbindungen der Formel (Ib)

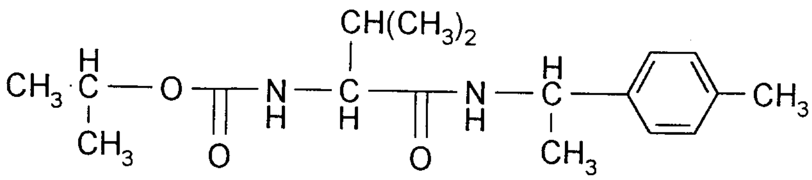 (Ib)

mit

(F) Mancozeb der Formel

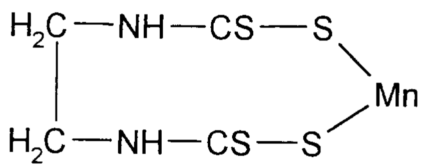 (VI)

**8.** Synergistisch wirkende Kombination von Verbindungen der Formel (Ib)

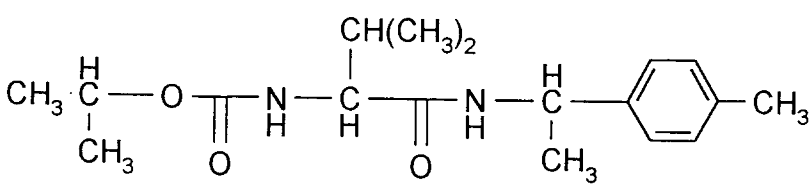 (Ib)

mit

(H) Kupfer-Oxychloride.

**9.** Synergistisch wirkende Kombination von Verbindungen der Formel (Ib)

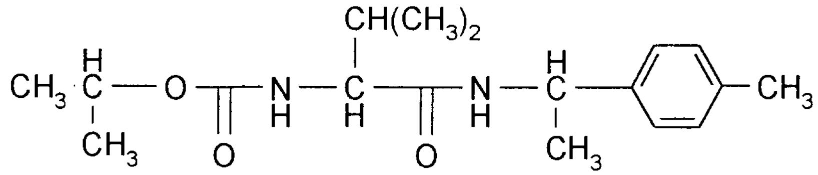

(Ib)

mit

(I) Captan der Formel

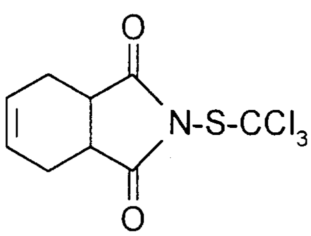

(VIII)

**10.** Synergistisch wirkende Kombination von Verbindungen der Formel (Ib)

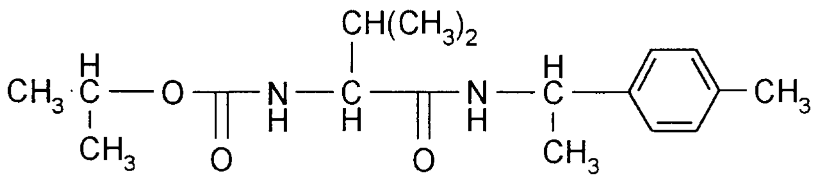

(Ib)

mit

(L) Dithianon der Formel

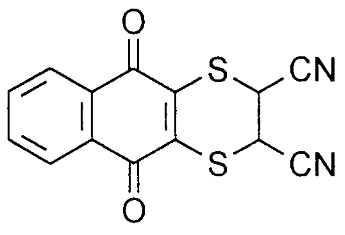

(X)

**11.** Synergistisch wirkende Kombination von Verbindungen der Formel (Ib)

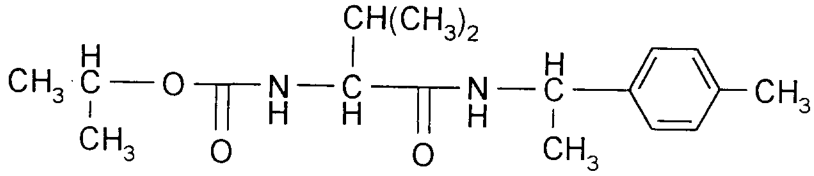

(Ib)

mit

(M) Phaltan der Formel

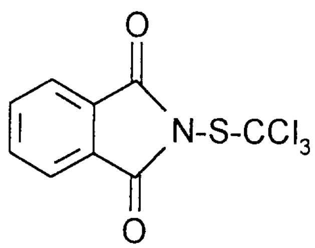

(XI)

**12.** Synergistisch wirkende Kombination von Verbindungen der Formel (Ib)

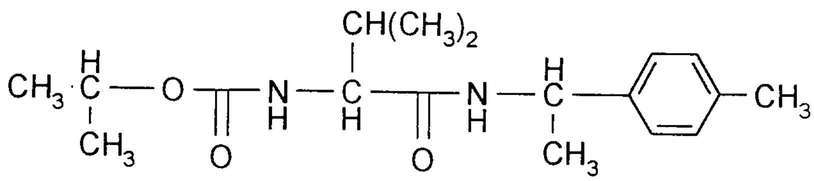

(Ib)

mit

(N) Cymoxanil der Formel

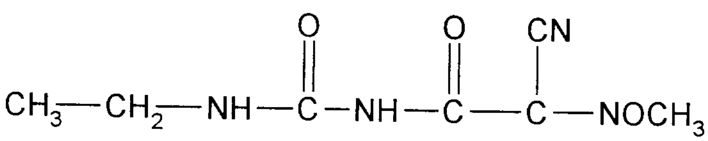

(XII)

**13.** Synergistisch wirkende Kombination von Verbindungen der Formel (Ib)

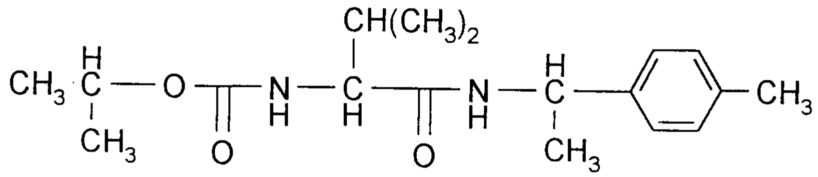 (Ib)

mit

(P) Fosetyl der Formel

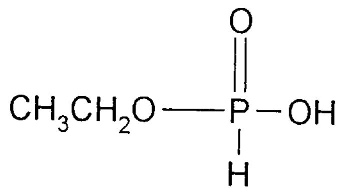 (XIV)

bzw. dessen Aluminium-Addukt

**14.** Synergistisch wirkende Kombination von Verbindungen der Formel (Ib)

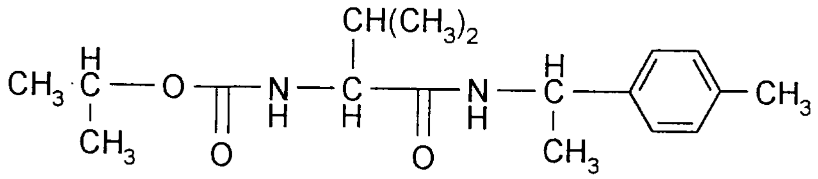 (Ib)

mit

(Q) Metalaxyl der Formel

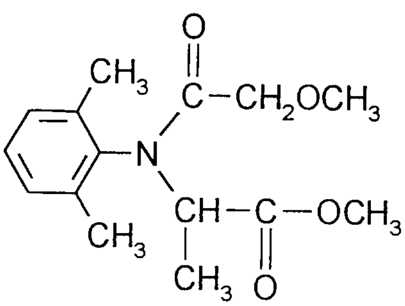 (XV)

**15.** Synergistisch wirkende Kombination von Verbindungen der Formel (Ib)

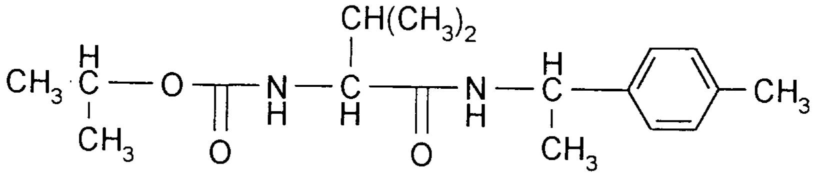

(Ib)

mit

(S) Fluazinam der Formel

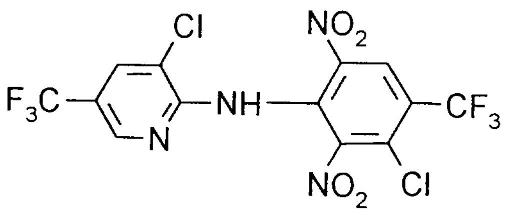

(XVII)

**16.** Synergistisch wirkende Kombination von Verbindungen der Formel (Ib)

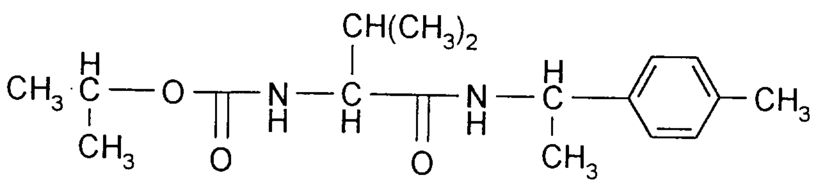

(Ib)

mit

(T) Methoxyacrylate wie
Methyl-(E)-2-{2-[6-(2-cyanophenoxy)pyrimidin-4-yloxy]phenyl}-3-methoxyacrylat der Formel

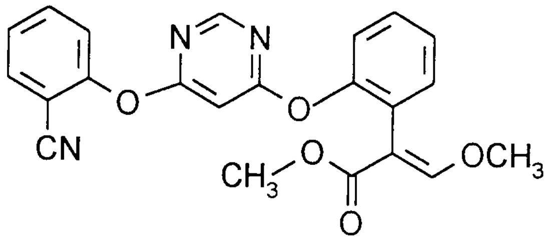

(XVIII)

**17.** Synergistisch wirkende Kombination von Verbindungen der Formel (Ib)

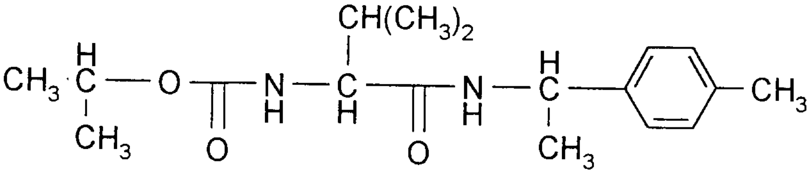

(Ib)

mit

(U) Methoximinoacetate wie
Methyl-(E)-methoximino[α-(o-tolyloxy)-o-tolyl]acetat der Formel

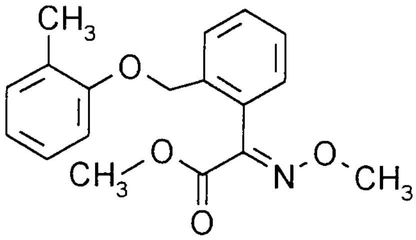

(XIX)

**18.** Synergistisch wirkende Kombination von Verbindungen der Formel (Ib)

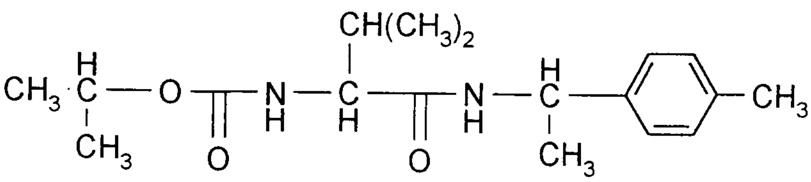

(Ib)

mit

(WI) Triadimenol.

**19.** Synergistisch wirkende Kombination von Verbindungen der Formel (Ib)

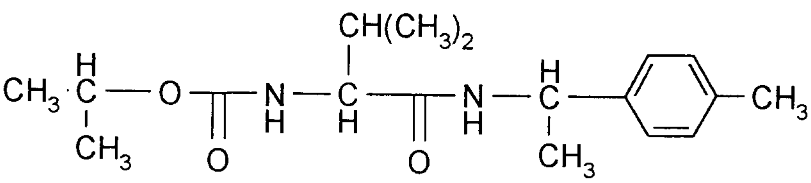

(Ib)

mit

(WII) Bitertanol.

**20.** Synergistisch wirkende Kombination von Verbindungen der Formel (Ib)

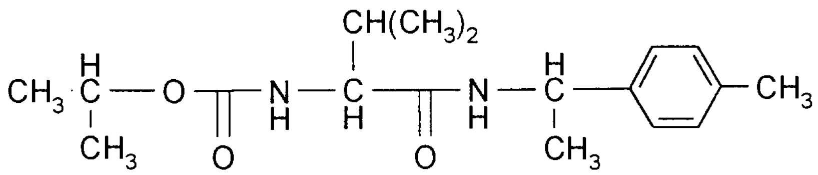 (Ib)

mit

(WIII) Triadimefon.

**21.** Synergistisch wirkende Kombination von Verbindungen der Formel (Ib)

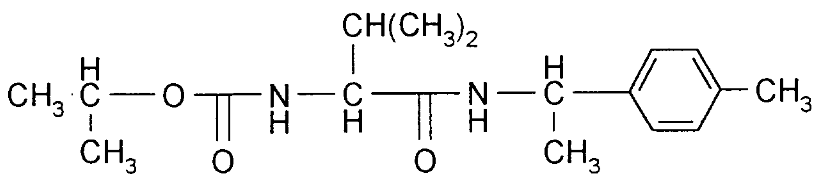 (Ib)

mit

(WIV) Tebuconazole.

**22.** Synergistisch wirkende Kombination von Verbindungen der Formel (Ib)

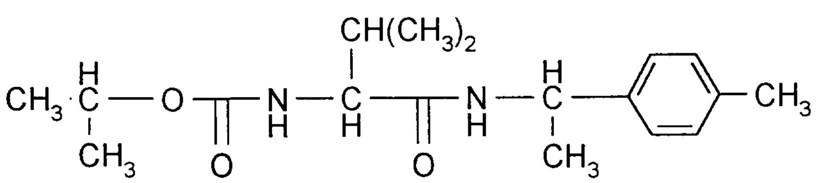 (Ib)

mit

(Y) Pencycuron.

**23.** Verfahren zur Bekämpfung von Pilzen, dadurch gekennzeichnet, daß man Wirkstoffkombinationen gemäß den Ansprüchen 1 bis 22 auf die Pilze und/oder deren Lebensraum einwirken läßt.

**24.** Verwendung von Wirkstoffkombinationen gemäß den Ansprüchen 1 bis 22 zur Bekämpfung von Pilzen.

**25.** Verfahren zur Herstellung von Mitteln, dadurch gekennzeichnet, daß man Wirkstoffkombinationen gemäß den Ansprüchen 1 bis 22 mit Streckmitteln und/oder oberflächenaktiven Stoffen vermischt.

**26.** Mittel enthaltend eine Wirkstoffkombination gemäß den Ansprüchen 1 bis 22.

**27.** Mittel gemäß Anspruch 1, dadurch gekennzeichnet, daß in den Wirkstoffkombinationen das Gewichtsverhältnis von Wirkstoff der Formel (I)

- zu Wirkstoff der Gruppe (A) zwischen 1:1 und 1:50 liegt,
- zu Wirkstoff der Gruppe (B) zwischen 1:1 und 1:50 liegt,
- zu Wirkstoff der Gruppe (C) zwischen 1:1 und 1:50 liegt,
- zu Wirkstoff der Gruppe (D) zwischen 1:1 und 1:50 liegt,

- zu Wirkstoff der Gruppe (E) zwischen 1:1 und 1:200 liegt,
- zu Wirkstoff der Gruppe (F) zwischen 1:1 und 1:50 liegt,
- zu Wirkstoff der Gruppe (G) zwischen 1:1 und 1:200 liegt,
- zu Wirkstoff der Gruppe (H) zwischen 1:1 und 1:200 liegt,
- zu Wirkstoff der Gruppe (I) zwischen 1:1 und 1:100 liegt,
- zu Wirkstoff der Gruppe (K) zwischen 1:0,5 und 1:10 liegt,
- zu Wirkstoff der Gruppe (L) zwischen 1:1 und 1:50 liegt,
- zu Wirkstoff der Gruppe (M) zwischen 1:1 und 1:50 liegt,
- zu Wirkstoff der Gruppe (N) zwischen 1:0,5 und 1:20 liegt,
- zu Wirkstoff der Gruppe (O) zwischen 1:1 und 1:100 liegt,
- zu Wirkstoff der Gruppe (P) zwischen 1:1 und 1:50 liegt,
- zu Wirkstoff der Gruppe (Q) zwischen 1:0,5 und 1:20 liegt,
- zu Wirkstoff der Gruppe (R) zwischen 1:0,5 und 1:20 liegt,
- zu Wirkstoff der Gruppe (S) zwischen 1:1 und 1:20 liegt,
- zu Wirkstoff der Gruppe (T) zwischen 1:0,5 und 1:20 liegt,
- zu Wirkstoff der Gruppe (U) zwischen 1:0,5 und 1:20 liegt.
- zu Wirkstoff der Gruppe (V) zwischen 1:0,5 und 1:20 liegt,
- zu Wirkstoff der Gruppe (W) zwischen 1:0,5 und 1:20 liegt,
- zu Wirkstoff der Gruppe (X) zwischen 1:0,5 und 1:20 liegt,
- zu Wirkstoff der Gruppe (Y) zwischen 1:0,5 und 1:20 liegt.

## Claims

**1.** Synergistically acting combination of compounds of the formula (I)

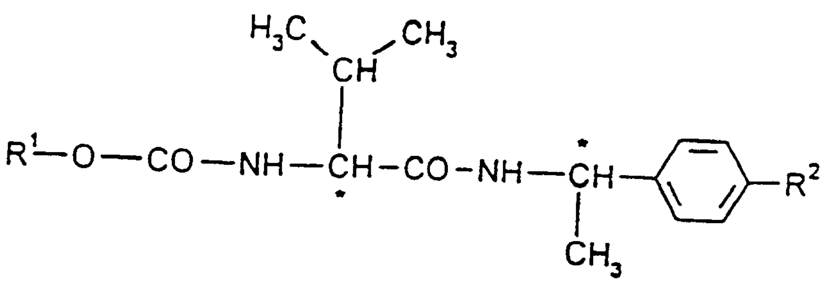

(I)

in which

$R^1$ represents i-propyl or s-butyl and

$R^2$ represents chlorine, methyl, ethyl or methoxy, and

(A) dichlofluanid, of the formula

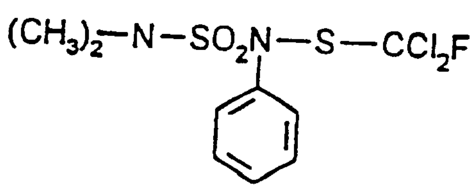

(II) ,

and/or

(B) tolylfluanid, of the formula

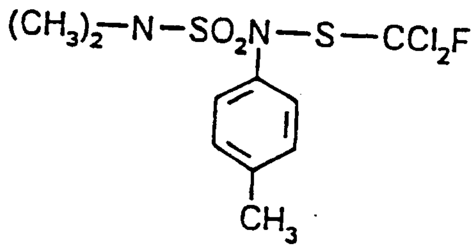

(III) ,

and/or

(C) tetrachloro-isophthalo-nitrile, of the formula

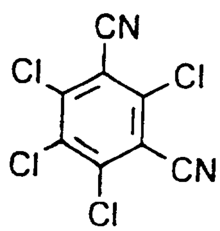

(IV) ,

(CHLOROTHALONIL)

and/or

(D) propineb, of the formula

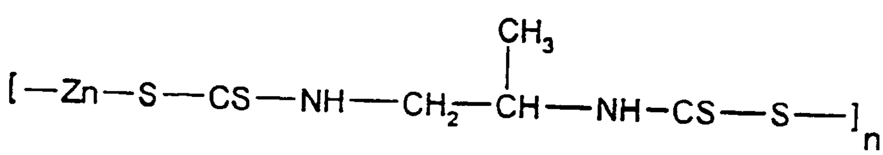

(V) ,

and/or

(E) tetramethyl-thiuram disulphide, of the formula

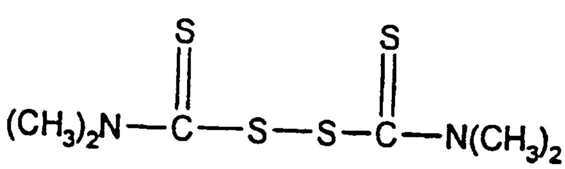

(VI) ,

(THIRAM)

and/or

(F) mancozeb, of the formula

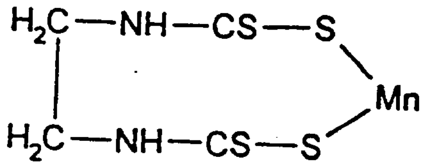

(VI) ,

and/or

(G) Dyrene, of the formula

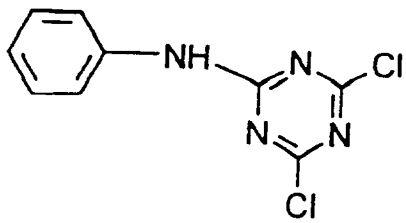

(VII) ,

and/or

(H) copper oxychloride
and/or

(I) captan, of the formula

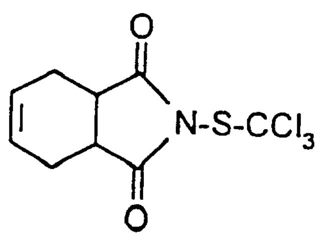

(VIII) ,

and/or

(K) a morpholine derivative of the formula

(dimethomorph)

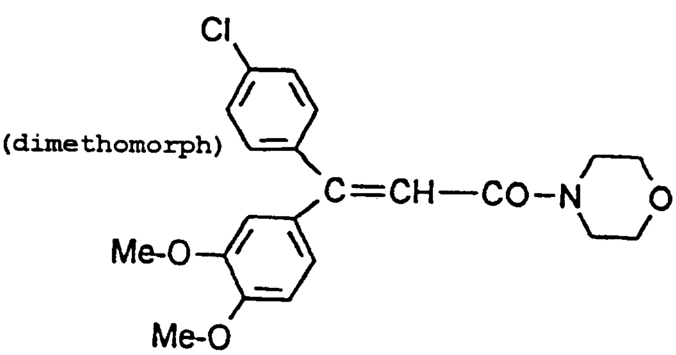

(IX) ,

and/or

(L) dithianon, of the formula

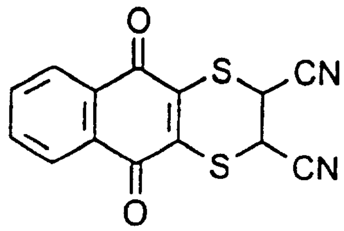

(X) ,

and/or

(M) phaltan, of the formula

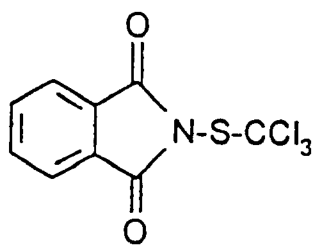

(XI) ,

and/or

(N) cymoxanil, of the formula

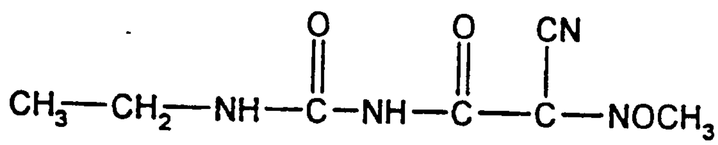

(XII) ,

and/or

(O) propamocarb, of the formula

$$(CH_3)_2NCH_2\text{-}CH_2\text{-}CH_2\text{-}NHCO\text{-}OCH_2CH_3 \quad (XIII),$$

or its hydrochloride
and/or

(P) fosetyl, of the formula

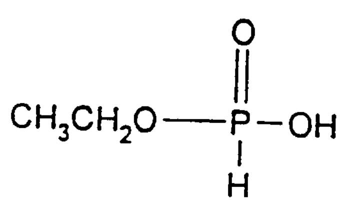

(XIV) ,

or its aluminium adduct
and/or

(Q) metalaxyl, of the formula

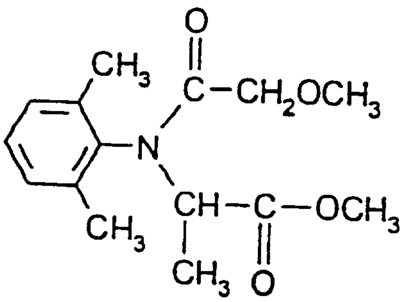

(XV) ,

and/or

(R) oxadixyl, of the formula

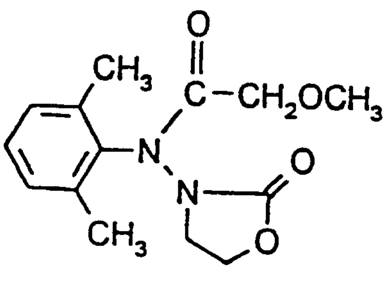

(XVI) ,

and/or

(S) fluazinam, of the formula

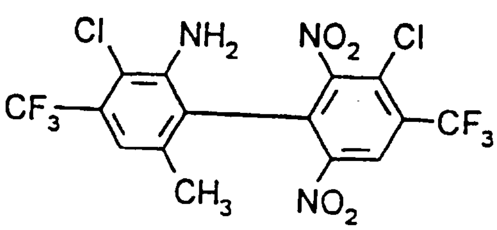

(XVII) ,

and/or

(T) methoxyacrylates, such as
methyl (E)-2-{2-[6-(2-cyanophenoxy)pyrimidin-4-yloxy]phenyl}-3-methoxyacrylate, of the formula

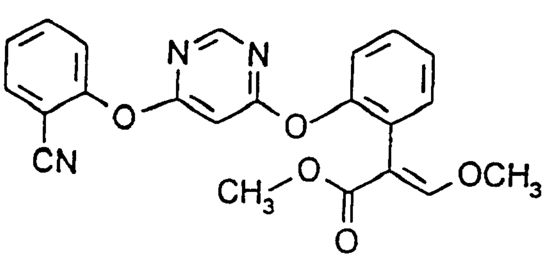

(XVIII) ,

and/or

(U) methoximinoacetates, such as
methyl (E)-methoximino[α-(o-tolyloxy)-o-tolyl]acetate, of the formula

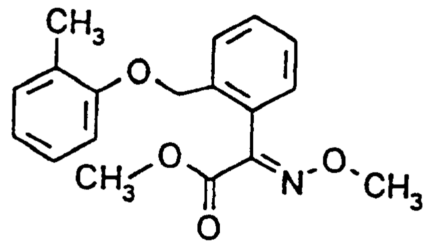

(XIX), ,

(V) furalaxyl (methyl N-(2-furoyl)-N-(2,6-xylyl)alaninate) and/or

(W) azoles of the formula

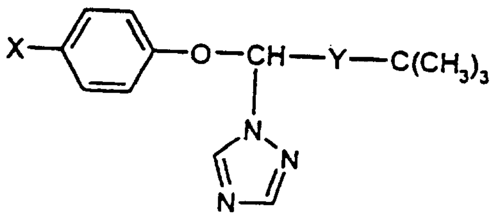

(XX) ,

I X = Cl, Y = -CH(OH)- (triadimenol)

II X = 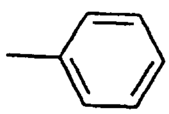 Y = -CH(OH)- (bitertanol)

III X = Cl, Y = -CO- (triadimefon)

IV tebuconazole, of the formula

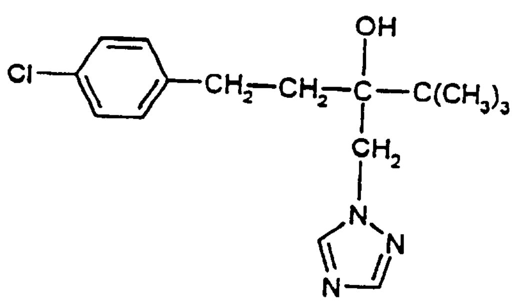

,

and/or

(X) etridiazole (3-trichloromethyl-5-ethoxy-1,2,4-thiadiazole) and/or

(Y) pencycuron (1-(4-chlorobenzyl)-1-cyclopentyl-3-phenylurea).

**2.** Synergistically acting combination of compounds of the formula (Ib)

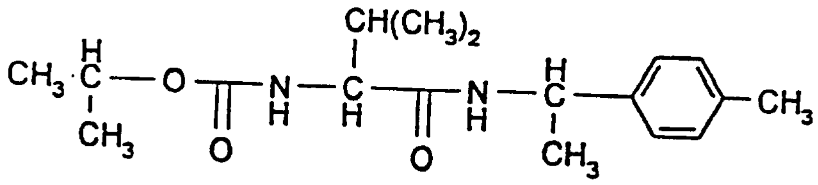

(Ib) ,

with

(A) dichlofluanid, of the formula (II)

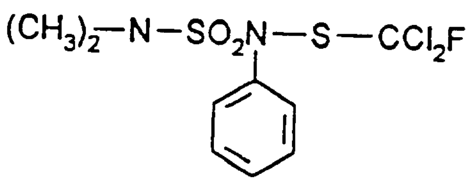

(II).

**3.** Synergistically acting combination of compounds of the formula (Ib)

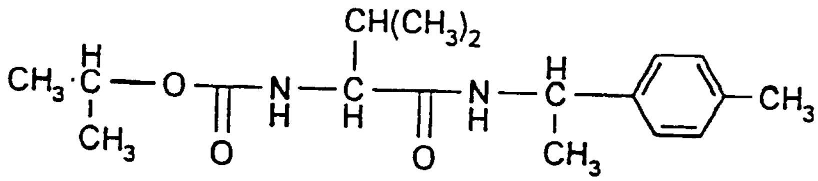

(Ib) ,

with

(B) tolylfluanid, of the formula

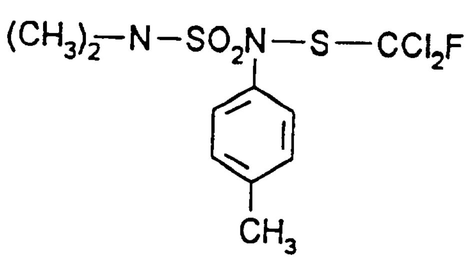

(III).

**4.** Synergistically acting combination of compounds of the formula (Ib)

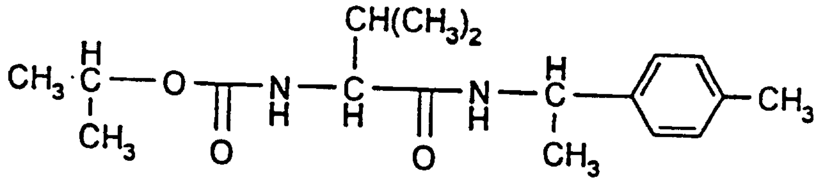

(Ib) ,

with

(C) tetrachloro-isophthalo-nitrile, of the formula

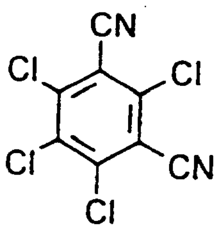

(IV)

(CHLOROTHALONIL)

**5.** Synergistically acting combination of compounds of the formula (Ib)

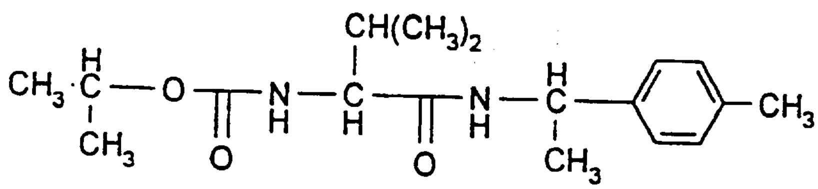

(Ib)

with

(D) propineb, of the formula

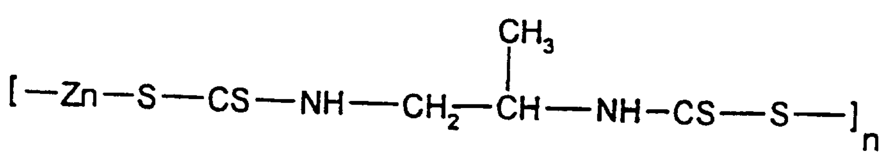

(V) .

**6.** Synergistically acting combination of compounds of the formula (Ib)

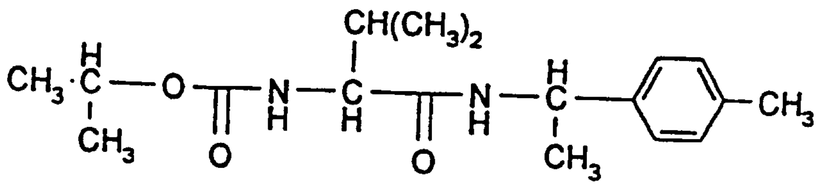

(Ib) ,

with

(E) tetramethyl-thiuram disulfide, of the formula

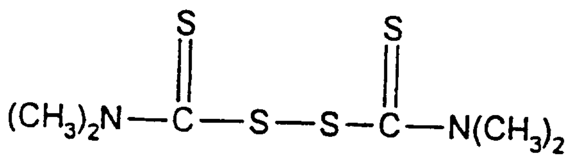

(VI) .

(THIRAM)

7. Synergistically acting combination of compounds of the formula (Ib)

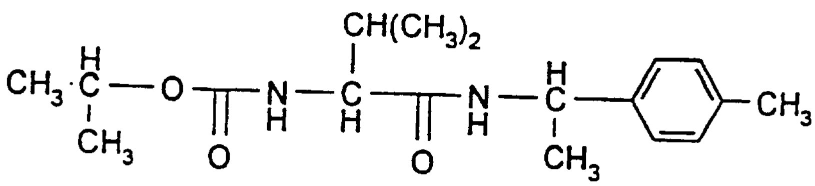

(Ib) ,

with

(F) mancozeb, of the formula

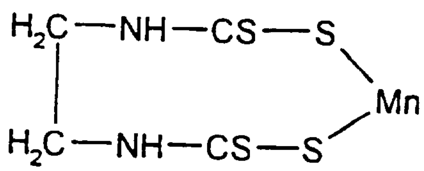

(VI) .

8. Synergistically acting combination of compounds of the formula (Ib)

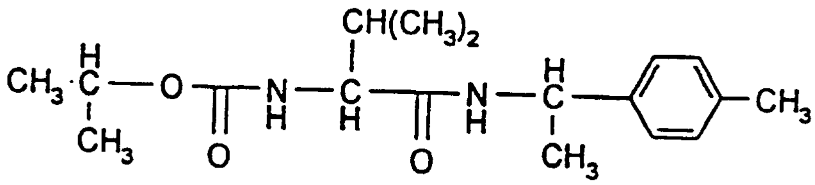

(Ib) ,

with

(H) copper oxychloride.

**9.** Synergistically acting combination of compounds of the formula (Ib)

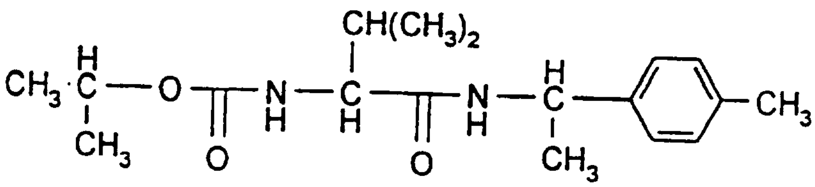

(Ib) ,

with

(I) captan, of the formula

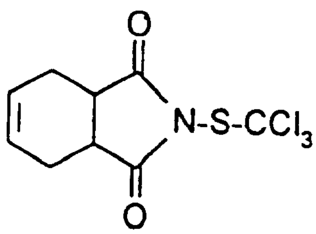

(VIII) .

**10.** Synergistically acting combination of compounds of the formula (Ib)

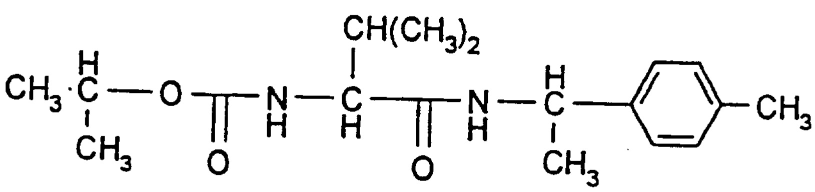

(Ib) ,

with

(L) dithianon, of the formula

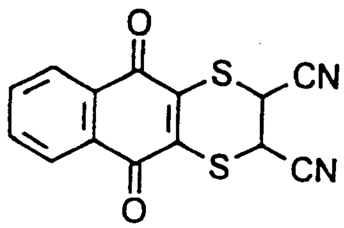

(X) .

**11.** Synergistically acting combination of compounds of the formula (Ib)

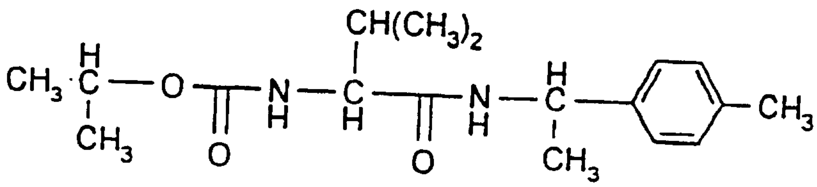

(Ib) ,

with

(M) phaltan, of the formula

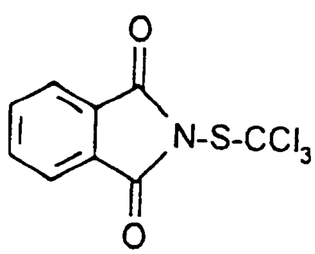

(XI) .

**12.** Synergistically acting combination of compounds of the formula (Ib)

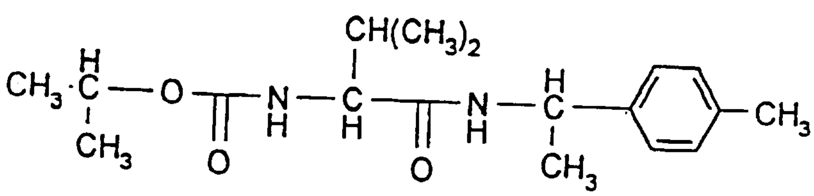

(Ib) ,

with

(N) cymoxanil, of the formula

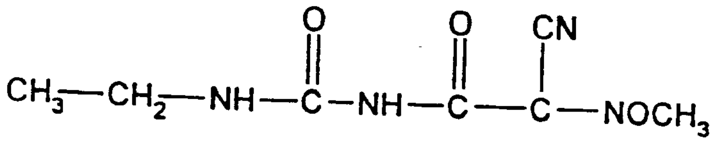

(XII) .

**13.** Synergistically acting combination of compounds of the formula (Ib)

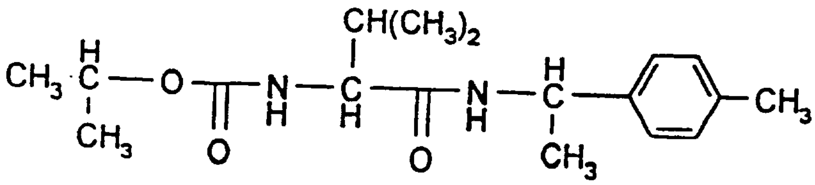

(Ib) ,

with

(P) fosetyl, of the formula

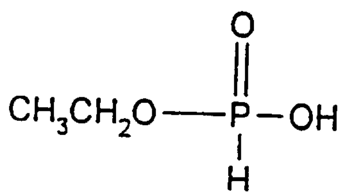

(XIV) ,

or its aluminium adduct.

**14.** Synergistically acting combination of compounds of the formula (Ib)

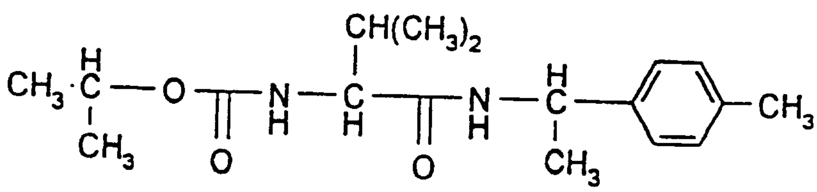

(Ib) ,

with

(Q) metalaxyl, of the formula

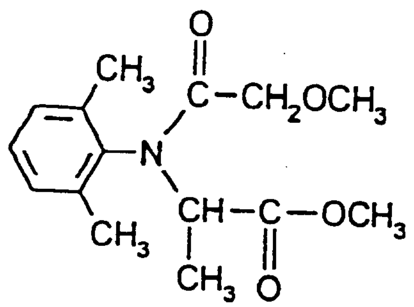

(XV) .

**15.** Synergistically acting combination of compounds of the formula (Ib)

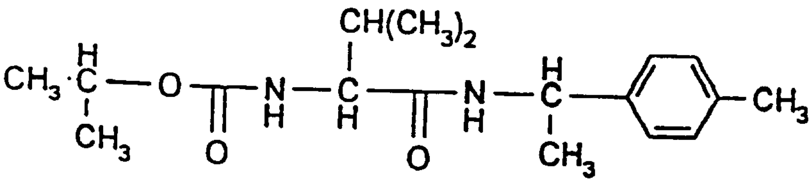

(Ib) ,

with

(S) fluazinam, of the formula

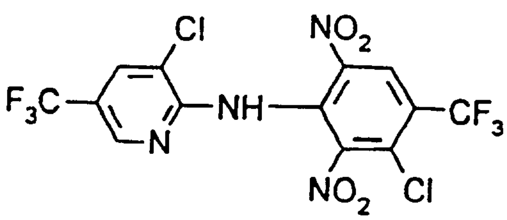

(XVII) .

**16.** Synergistically acting combination of compounds of the formula (Ib)

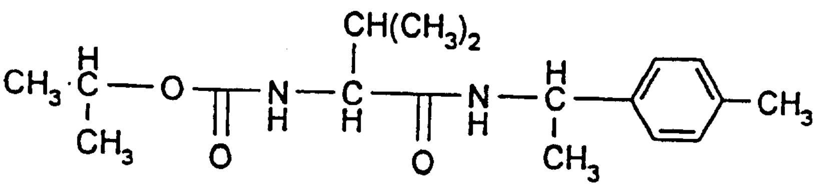

(Ib) ,

with

(T) methoxyacrylates, such as

methyl (E)-2-{2-[6-(2-cyanophenoxy)pyrimidin-4-yloxy]phenyl}-3-methoxyacrylate, of the formula

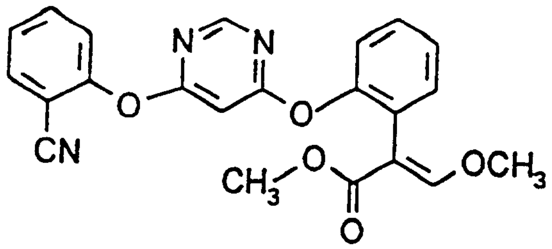

(XVIII) .

**17.** Synergistically acting combination of compounds of the formula (Ib)

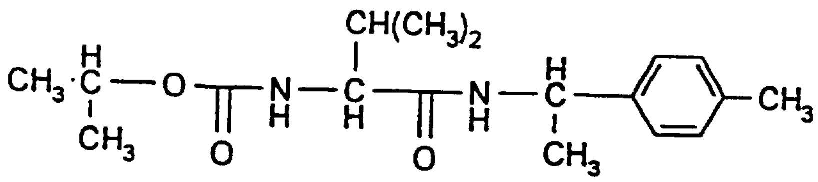

(Ib) ,

with

(U) methoximinoacetates, such as
methyl (E)-methoximino[α-(o-tolyloxy)-o-tolyl]acetate, of the formula

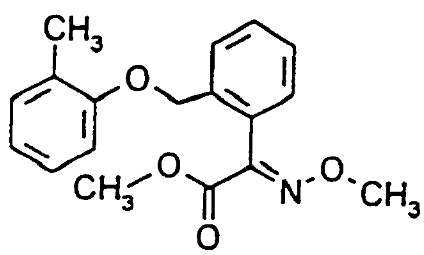

(XIX) .

**18.** Synergistically acting combination of compounds of the formula (Ib)

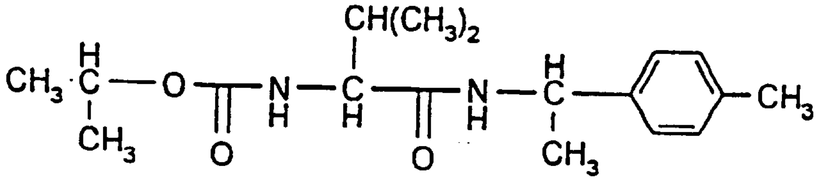

(Ib) ,

with

(WI) triadimenol.

**19.** Synergistically acting combination of compounds of the formula (Ib)

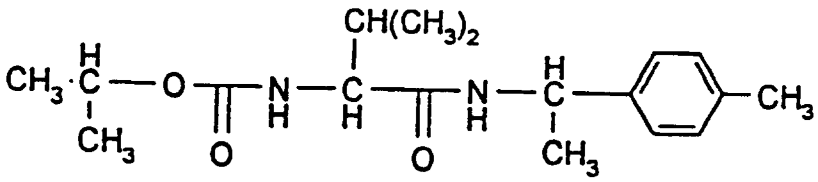

(Ib) ,

with

(WII) bitertanol.

**20.** Synergistically acting combination of compounds of the formula (Ib)

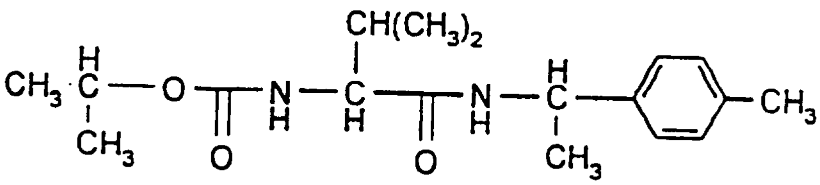

(Ib) ,

with

(WILL) triadimefon.

**21.** Synergistically acting combination of compounds of the formula (Ib)

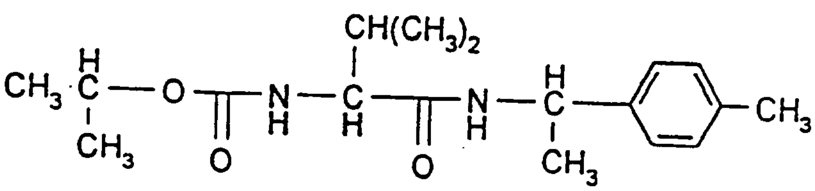

(Ib) ,

with

(WIV) tebuconazole.

**22.** Synergistically acting combination of compounds of the formula (Ib)

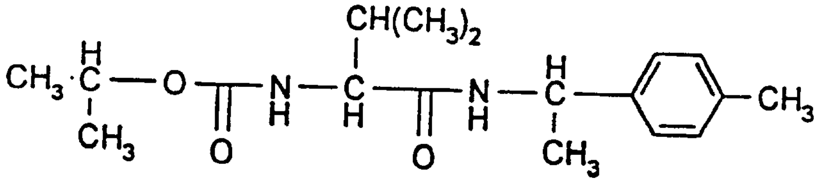

(Ib) ,

with

(Y) pencycuron.

**23.** Method of controlling fungi, characterized in that active substance combinations according to Claims 1 to 22 are allowed to act on the fungi and/or their environment.

**24.** Use of active substance combinations according to Claims 1 to 22 for controlling fungi.

**25.** Process for the preparation of compositions, characterized in that active substance combinations according to Claims 1 to 22 are mixed with extenders and/or surfactants.

**26.** Composition comprising an active substance combination according to Claims 1 to 22.

**27.** Combination according to Claim 1, characterized in that, in the active substance combinations, the weight ratio of active substance of the formula (I)

- to active substance of group (A) is between 1:1 and 1:50,
- to active substance of group (B) is between 1:1 and 1:50,
- to active substance of group (C) is between 1:1 and 1:50,
- to active substance of group (D) is between 1:1 and 1:50,
- to active substance of group (E) is between 1:1 and 1:200,
- to active substance of group (F) is between 1:1 and 1:50,
- to active substance of group (G) is between 1:1 and 1:200,
- to active substance of group (H) is between 1:1 and 1:200,
- to active substance of group (I) is between 1:1 and 1:100,
- to active substance of group (K) is between 1:0.5 and 1:10,
- to active substance of group (L) is between 1:1 and 1:50,
- to active substance of group (M) is between 1:1 and 1:50,
- to active substance of group (N) is between 1:0.5 and 1:20,
- to active substance of group (O) is between 1:1 and 1:100,
- to active substance of group (P) is between 1:1 and 1:50,
- to active substance of group (Q) is between 1:0.5 and 1:20,
- to active substance of group (R) is between 1:0.5 and 1:20,
- to active substance of group (S) is between 1:1 and 1:20,
- to active substance of group (T) is between 1:0.5 and 1:20,
- to active substance of group (U) is between 1:0.5 and 1:20,
- to active substance of group (V) is between 1:0.5 and 1:20,
- to active substance of group (W) is between 1:0.5 and 1:20,
- to active substance of group (X) is between 1:0.5 and 1:20,
- to active substance of group (Y) is between 1:0.5 and 1:20,

## Revendications

**1.** Combinaison à action synergique de composés de formule (I)

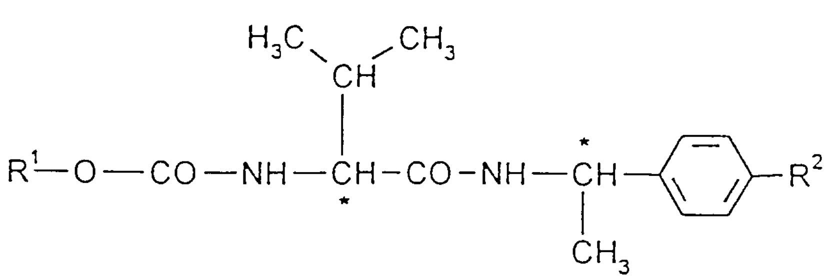

(I)

dans laquelle

$R^1$ est un groupe isopropyle ou sec.butyle et

$R^2$ représente du chlore, un groupe méthyle, éthyle ou méthoxy, avec

(A) le dichlofluanid de formule

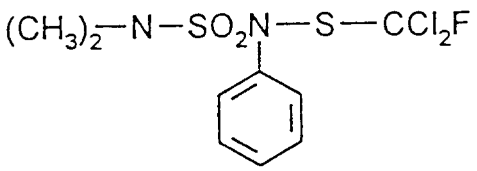

(II)

et/ou

(B) le tolylfluanid de formule

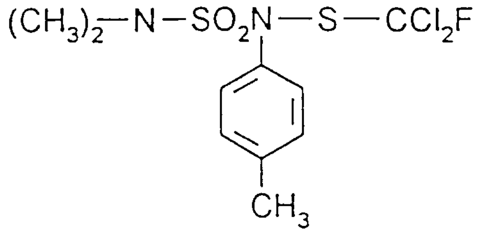

(III)

et/ou

(C) le tétrachlorisophtalo-dinitrile de formule

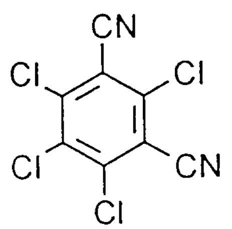

(IV)

(CHLOROTHALONIL)

et/ou

(D) le propinèbe de formule

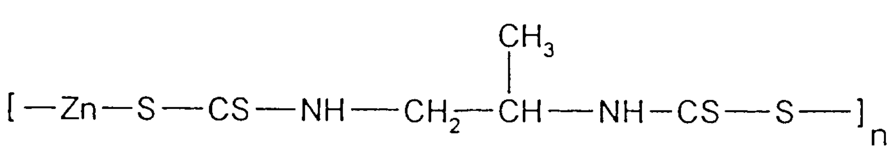

(V)

et/ou

(E) le disulfure de tétraméthyl-thiurame de formule

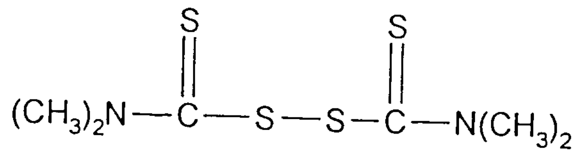

(VI)

(THIRAME)

et/ou

(F) le mancozèbe de formule

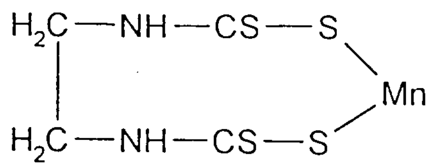

(VI)

et/ou

(G) le dyrène de formule

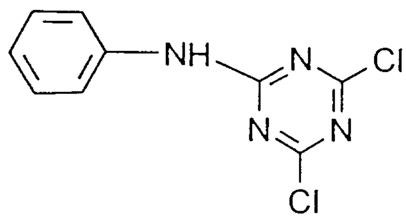

(VII)

et/ou

(H) des oxychlorures de cuivre

et/ou

(I) le captan de formule

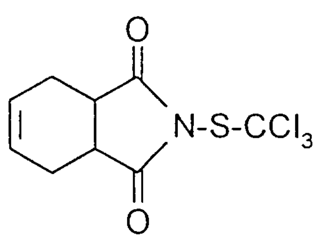

(VIII)

et/ou

(K) un dérivé de morpholine de formule

(Dimétomorphe)

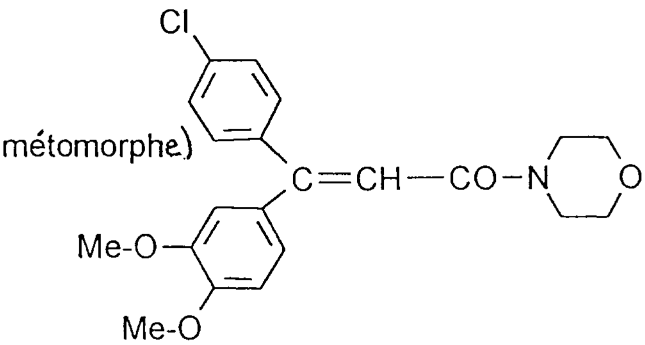

(IX)

et/ou

(L) le dithianon de formule

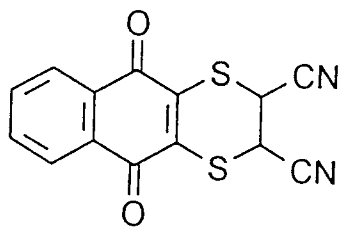

(X)

et/ou

(M) le phaltan de formule

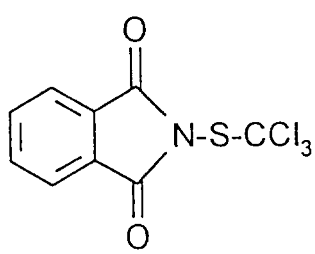

(XI)

et/ou

(N) le cymoxanil de formule

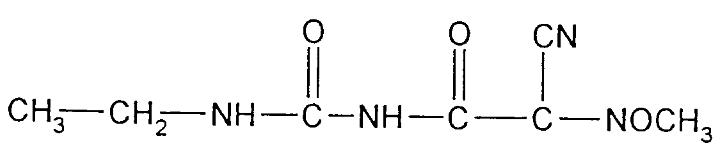

(XII)

et/ou

(O) le propamocarbe de formule

$$(CH_3)_2NCH_2\text{-}CH_2\text{-}CH_2\text{-}NHCO\text{-}OCH_2CH_3 \qquad (XIII)$$

ou son chlorhydrate et/ou

(P) le fosétyl de formule

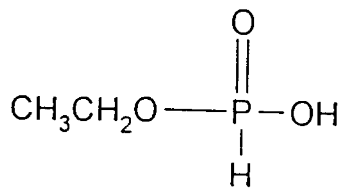

(XIV)

ou son produit d'addition avec l'aluminium

et/ou

(Q) le métalaxyl de formule

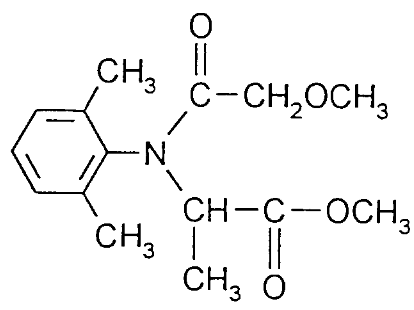

(XV)

et/ou

(R) l'oxadixyl de formule

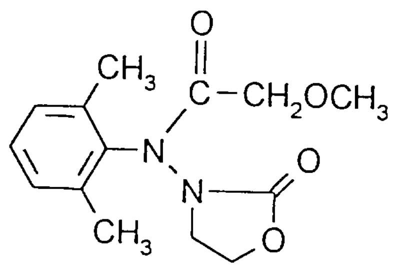

(XVI)

et/ou

(S) le fluazinam de formule

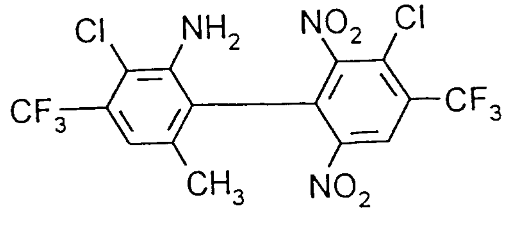

(XVII)

et/ou

(T) des méthoxyacrylates tels que le (E)-2-{2-[6(2-cyanophénoxy)pyrimidine-4-yloxy]phényl}-3-méthoxyacrylate de méthyle de formule

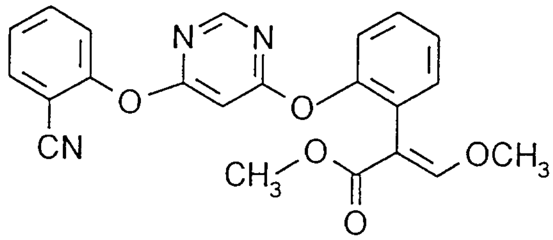

(XVIII)

et/ou

(U) des méthoximinoacétates tels que le (E)-méthoximino[α(o-tolyloxy)-o-tolyl]acétate de méthyle de formule

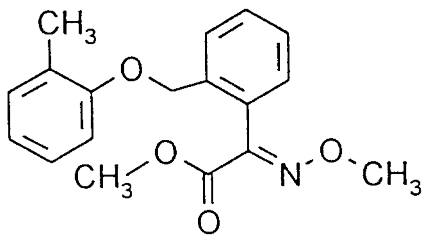

(XIX),

(V) le furalaxyl ≙ méthyl-N-(2-furoyl)-N-(2,6-xylyl)-alanite
et/ou
(W) des azoles de formule

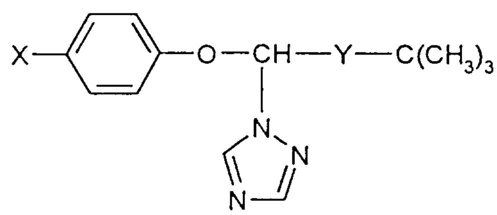

(XX)

I X = Cl, Y = -CH(OH)- (Triadiménol)

II X = 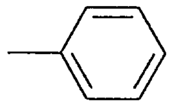 , Y = -CH(OH)- (Bitertanol)

III X = Cl, Y = -CO- (Triadiméfon)

IV le tébuconazole de formule

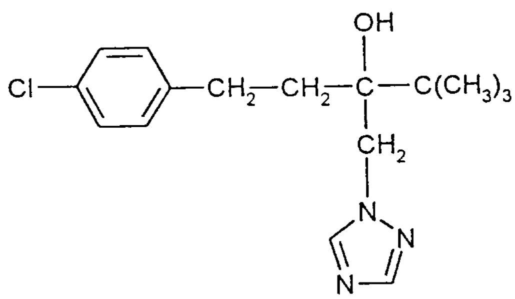

et/ou
(X) l'étridiazole ≙ 3-trichlorométhyl-5-éthoxy-1,2,4-thiadiazole
et/ou
(Y) le pénaycuron ≙ 1-(4-chlorobenzyl)-1-cyclopentyl-3-phénylurée.

**2.** Combinaison à action synergique de composés de formule (Ib)

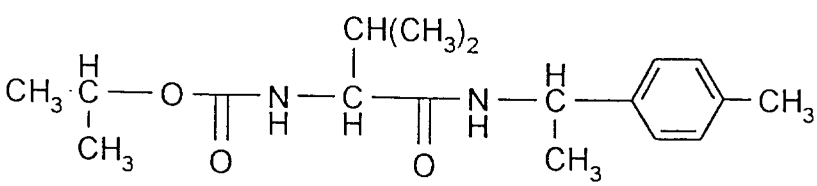 (Ib)

avec

(A) le dichlofluanid de formule (II)

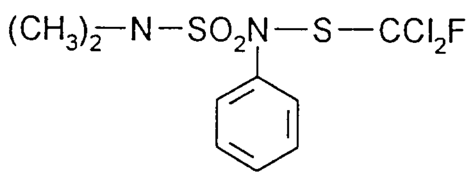 (II).

**3.** Combinaison à action synergique de composés de formule (Ib)

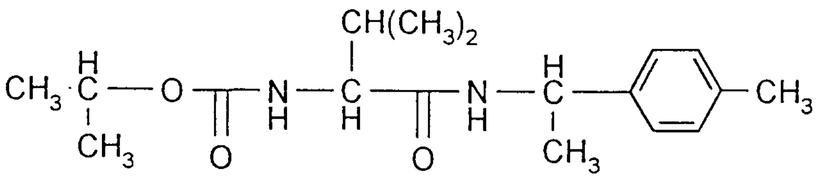

(Ib)

avec

(B) le tolylfluanid de formule

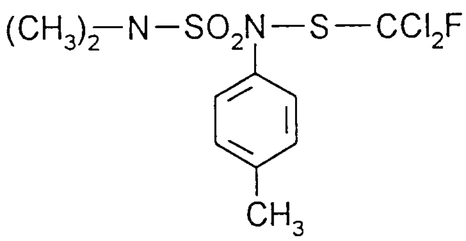

(III).

**4.** Combinaison à action synergique de composés de formule (Ib)

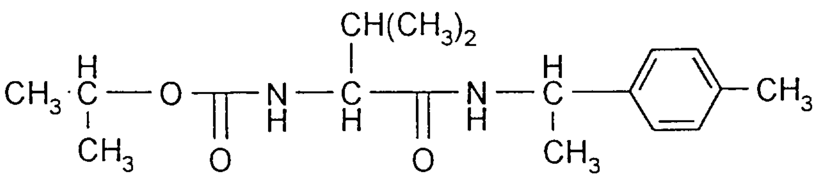

(Ib)

avec

(C) le tétrachlorisophtalo-dinitrile de formule

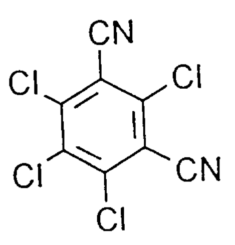

(IV)

(CHLOROTHALONIL)

**5.** Combinaison à action synergique de composés de formule (Ib)

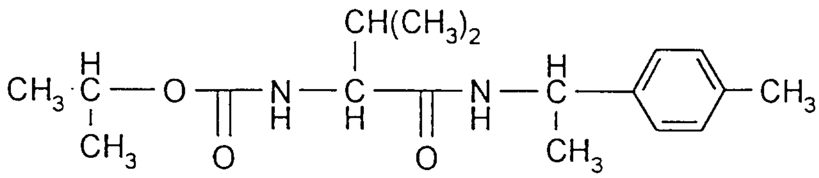

(Ib)

avec

(D) le propinèbe de formule

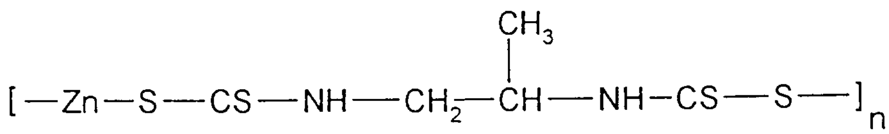

(V)

6. Combinaison à action synergique de composés de formule (Ib)

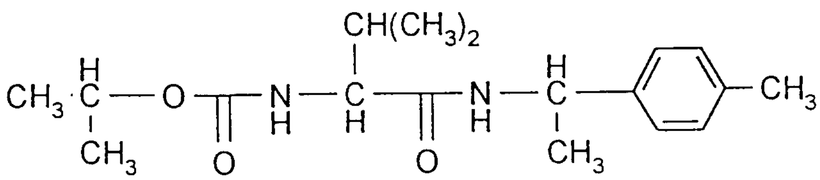

(Ib)

avec

(E) le disulfure de tétraméthyl-thiurame de formule

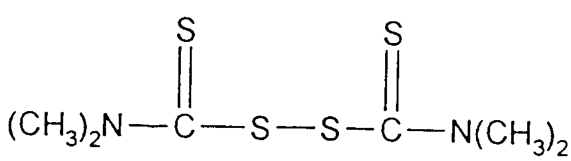

(VI)

(THIRAME)

7. Combinaison à action synergique de composés de formule (Ib)

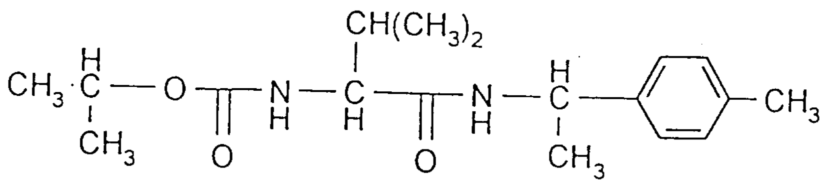

(Ib)

avec

(F) le mancozèbe de formule

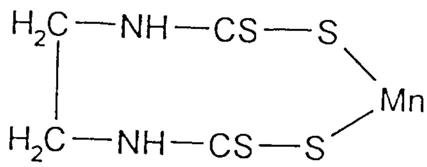

(VI)

**8.** Combinaison à action synergique de composés de formule (Ib)

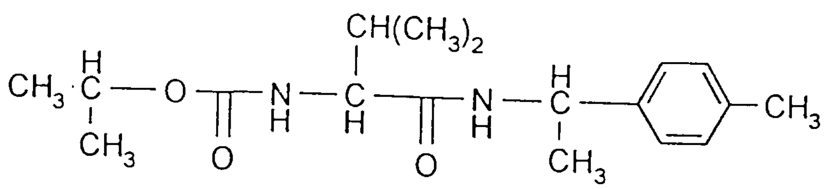

(Ib)

avec

(H) des oxychlorures de cuivre

**9.** Combinaison à action synergique de composés de formule (Ib)

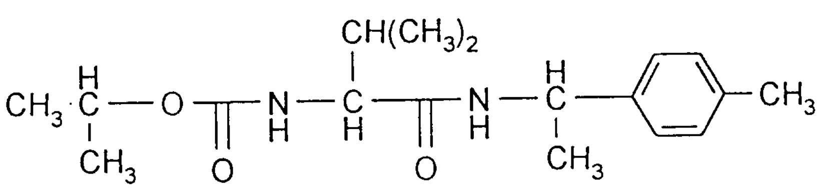

(Ib)

avec

(I) le captan de formule

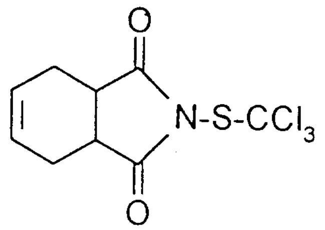

(VIII)

**10.** Combinaison à action synergique de composés de formule (Ib)

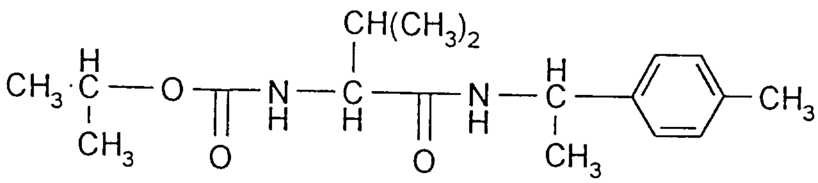

(Ib)

avec

(L) le dithianon de formule

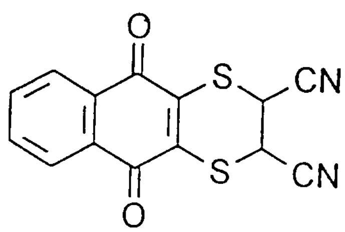

(X)

**11.** Combinaison à action synergique de composés de formule (Ib)

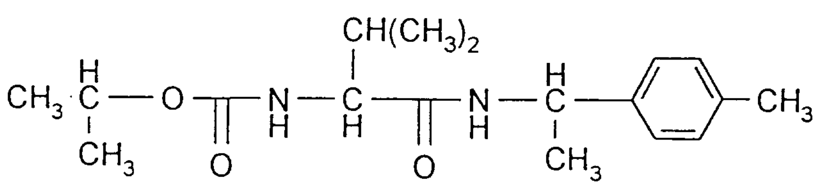

(Ib)

avec

(M) le phaltan de formule

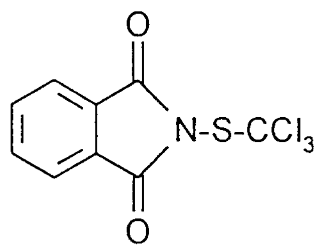

(XI)

**12.** Combinaison à action synergique de composés de formule (Ib)

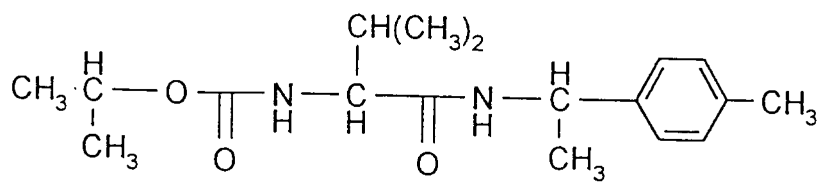

(Ib)

avec

(N) le cymoxanil de formule

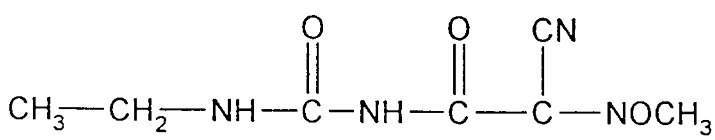

(XII)

**13.** Combinaison à action synergique de composés de formule (Ib)

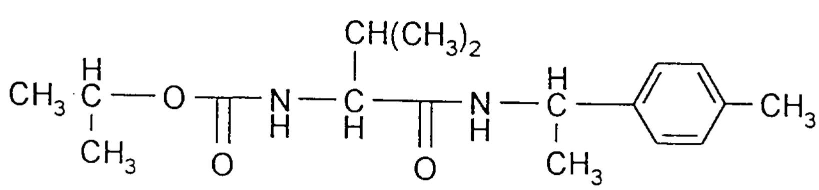

(Ib)

avec

(P) le fosétyl de formule

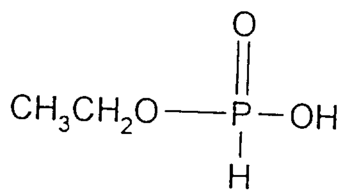

(XIV)

ou son produit d'addition avec l'aluminium

**14.** Combinaison à action synergique de composés de formule (Ib)

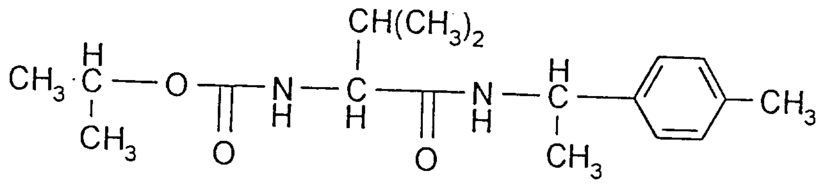

(Ib)

avec

(Q) le métalaxyl de formule

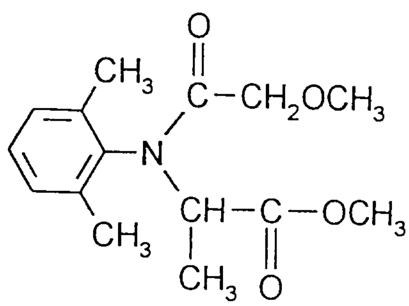

(XV)

**15.** Combinaison à action synergique de composés de formule (Ib)

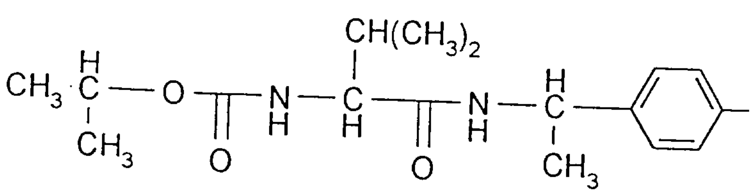

avec

(S) le fluzinam de formule

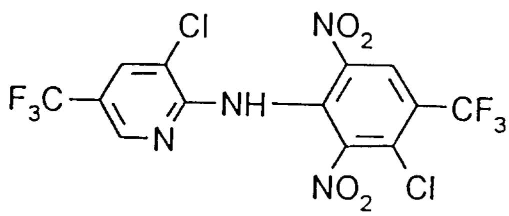

(XVII)

**16.** Combinaison à action synergique de composés de formule (Ib)

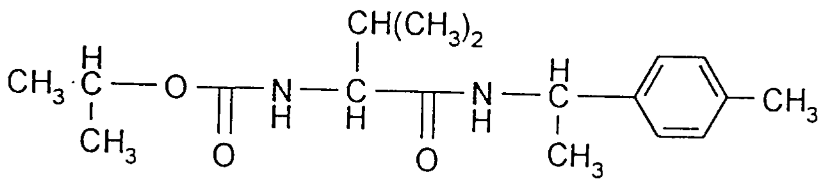

(Ib)

avec

(T) des méthoxyacrylates tels que le (E)-2-{2-[6(2-cyanophénoxy)pyrimidine-4-yloxy]phényl}-3-méthoxyacrylate de méthyle de formule

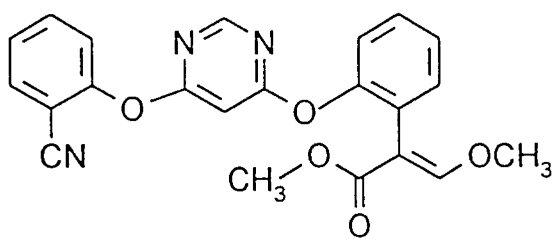

(XVIII)

**17.** Combinaison à action synergique de composés de formule (Ib)

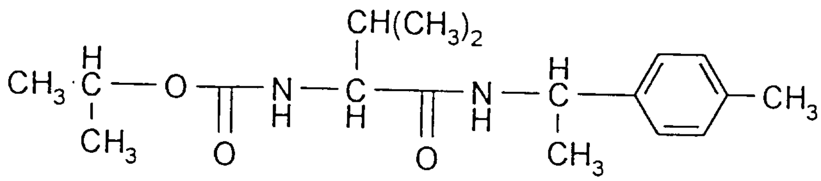

(Ib)

avec

(U) des méthoximinoacétates tels que le (E)-méthoximino[α-(o-tolyloxy)-o-tolyl]acétate de méthyle de formule

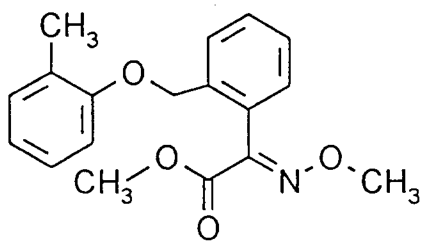

(XIX)

**18.** Combinaison à action synergique de composés de formule (Ib)

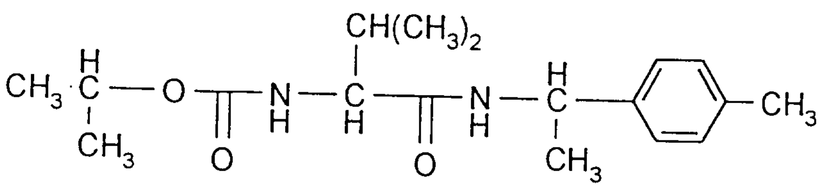

(Ib)

avec

(WI) le triadiménol.

**19.** Combinaison à action synergique de composés de formule (Ib)

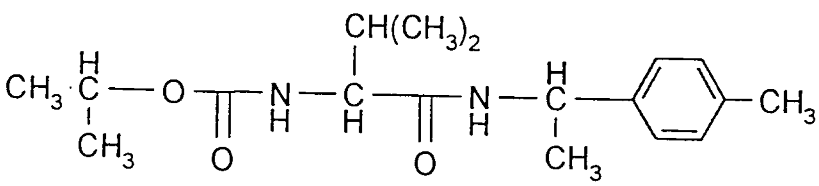

(Ib)

avec

(WII) le bitertanol.

**20.** Combinaison à action synergique de composés de formule (Ib)

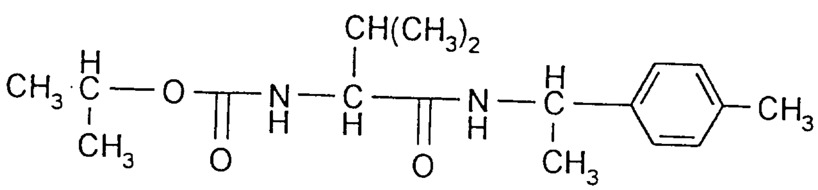

(Ib)

avec

(WIII) le triadiméfon.

**21.** Combinaison à action synergique de composés de formule (Ib)

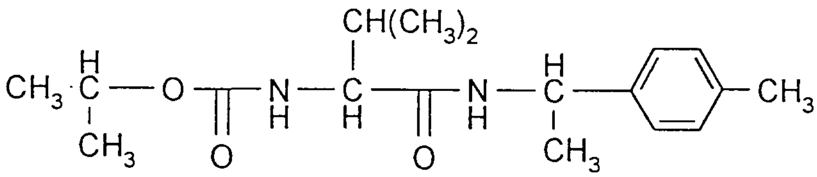

(Ib)

avec

(WIV) le tébuconazole.

**22.** Combinaison à action synergique de composés de formule (Ib)

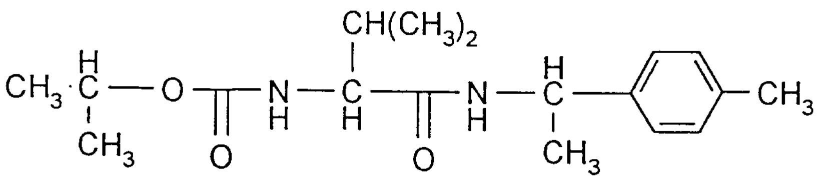

(Ib)

avec (Y) le pencycuron.

**23.** Procédé pour combattre des champignons, caractérisé en ce qu'on fait agir des combinaisons de substances actives suivant les revendications 1 à 22 sur les champignons et/ou sur leur milieu.

**24.** Utilisation de combinaisons de substances actives suivant les revendications 1 à 22 pour combattre des champignons.

**25.** Procédé de préparation de compositions, caractérisé en ce qu'on mélange des combinaisons de substances actives suivant les revendications 1 à 22 avec des diluants et/ou des agents tensio-actifs.

**26.** Composition contenant une combinaison de substances actives selon les revendications 1 à 22.

**27.** Combinaison suivant la revendication 1, caractérisée en ce que dans les combinaisons de substances actives, le rapport en poids de la substance active de formule (I)

- avec la substance active du groupe (A) se situe entre 1:1 et 1:50,
- avec la substance active du groupe (B) se situe entre 1:1 et 1:50,
- avec la substance active du groupe (C) se situe entre 1:1 et 1:50,
- avec la substance active du groupe (D) se situe entre 1:1 et 1:50,
- avec la substance active du groupe (E) se situe entre 1:1 et 1:200,
- avec la substance active du groupe (F) se situe entre 1:1 et 1:50,
- avec la substance active du groupe (G) se situe entre 1:1 et 1:200,
- avec la substance active du groupe (H) se situe entre 1:1 et 1:200,
- avec la substance active du groupe (I) se situe entre 1:1 et 1:100,
- avec la substance active du groupe (K) se situe entre 1:0,5 et 1:10,
- avec la substance active du groupe (L) se situe entre 1:1 et 1:50,
- avec la substance active du groupe (M) se situe entre 1:1 et 1:50,
- avec la substance active du groupe (N) se situe entre 1:0,5 et 1:20,
- avec la substance active du groupe (O) se situe entre 1:1 et 1:100,
- avec la substance active du groupe (P) se situe entre 1:1 et 1:50,
- avec la substance active du groupe (Q) se situe entre 1:0,5 et 1:20,

- avec la substance active du groupe (R) se situe entre 1:0,5 et 1:20,
- avec la substance active du groupe (S) se situe entre 1:1 et 1:20,
- avec la substance active du groupe (T) se situe entre 1:0,5 et 1:20,
- avec la substance active du groupe (U) se situe entre 1:0,5 et 1:20,
- avec la substance active du groupe (V) se situe entre 1:0,5 et 1:20,
- avec la substance active du groupe (W) se situe entre 1:0,5 et 1:20,
- avec la substance active du groupe (X) se situe entre 1:0,5 et 1:20,
- avec la substance active du groupe (Y) se situe entre 1:0,5 et 1:20,